# EUROPEAN PATENT SPECIFICATION

(11) **EP 2 191 853 B1**
(45) Date of publication and mention of the grant of the patent: **29.07.2015**
(21) Application number: 08833465.1
(22) Date of filing: 29.09.2008
(51) Int. Cl.: A61L 29/16, A61L 31/10, A61L 29/10, A61L 31/16, A61F 2/91

(54) **IN-VIVO INDWELLING MATTER**
IN-VIVO-DAUERIMPLANTATIONSMATERIAL
PRODUIT IMPLANTABLE IN VIVO

(30) Priority: 28.09.2007 JP 2007254540; 31.03.2008 JP 2008089451
(43) Date of publication of application: 02.06.2010
(73) Proprietor: Terumo Kabushiki Kaisha, Tokyo 151-0072 (JP)
(72) Inventor: NAKAGAWA, Yuji, Ashigarakami-gun Kanagawa 259-0151 (JP); SHIMURA, Kenichi, Ashigarakami-gun Kanagawa 259-0151 (JP)
(74) Representative: Prüfer & Partner GbR European Patent Attorneys
(86) International application number: PCT/JP2008/067672
(87) International publication number: WO 2009/041691

(56) References cited:
- WO-A1-03/039612
- WO-A1-2006/036967
- WO-A2-2007/047781
- JP-A- 1 124 463
- JP-A- 11 323 113
- JP-A- 2003 147 180
- JP-A- 2007 097 706
- JP-A- 2008 220 811
- JP-A- 2008 220 811
- US-A1- 2004 215 336
- DATABASE WPI Week 200873 Thomson Scientific, London, GB; AN 2008-M41364 XP002598395 & JP 2008 220811 A (TERUMO CORP) 25 September 2008 (2008-09-25)

## Description

### Technical Field

The present invention relates to a stent as an in-vivo indwelling device for delivering a medicinal agent to a lesion to achieve local administration in order to cure a stenosed or occluded lesion generated in a lumen in vivo, and a stent as an in-vivo indwelling device to be put indwelling in a stenosed or occluded lesion generated in a lumen in vivo so as to maintain patency of the lumen.

### Background Art

In recent years, in order to ameliorate a stenosed lesion generated in a lumen in vivo such as blood vessel, bile duct, trachea, esophagus, urethra, etc., a treatment called PTA (Percutaneous Transluminal Angioplasty) has been conducted in which the stenosed lesion is dilated into patency by a balloon catheter or a stent. A stent is a hollow tubular medical device which, for curing various diseases arising from stenosis or occlusion of a blood vessel or other lumen, can be put indwelling in the stenosed or occluded lesion to dilate the lesion and maintain patency of the lumen. Particularly, in the case where the curing procedure is applied to a stenosed or occluded lesion generated in a coronary artery of a heart, the procedure is called PTCA (Percutaneous Transluminal coronary Angioplasty) or PCI (Percutaneous Transluminal coronarylntervention), which is a well-established curing method. For instance, in relation to the coronary artery of the heart, this treatment procedure is performed for the purpose of preventing restenosis after PTCA.

In the early period of PCI, the procedure was conducted by using only a balloon catheter (Plain Old Balloon Angioplasty: POBA). However, the POBA in which the curing mechanism relies on forming a crack in a blood vessel and dilating the blood vessel often caused dissection of the coronary artery or recoiling. Therefore, the POBA was an unreliable curing method that frequently causes acute coronary occlusion or restenosis. Especially, the restenosis rate after POBA was as high as 40 to 50%. As a countermeasure against this problem, the hollow tubular medical device called stent has come to be put to clinical use. By putting a stent indwelling in a blood vessel, suppression of the acute coronary occlusion has come to be achieved successfully. However, restenosis is still observed in a rate of 20 to 30%, and the restenosis is a considerable problem yet to be solved. It was the advent of drug eluting stent (DES) that brought about a change in the circumstances. A DES is a device in which a medicinal agent such as immunosuppressor or carcinostatic agent is carried on a stent, and the DES is so set as to locally and sustainedly release the medicinal agent at the stenosed or occluded lesion in a lumen. Owing to the DES, the restenosis rate has been remarkably lowered to 10% or below.

For example, in Patent Document 1, a coating layer having a carcinostatic agent contained in a polyolefin elastomer is carried on a stent, thereby realizing local and sustained release of the biologically active agent. In Patent Document 2, sustained release of an immunosuppressor is achieved by using an acrylate polymer as a drug carrying polymer. Such a polymer for carrying the biologically active agent therein is characterized by its stability in vivo and its being endurable to deterioration such as cracking after the stent is put indwelling in a blood vessel. However, the polymers just mentioned are non-biodegradable polymers, so that they remain in the blood vessel after the disappearance of the biologically active agent. According to Non-patent Document 1, these polymers are considerably irritant in the manner of causing inflammation, so that the polymers are considered to constitute one of the causes of restenosis or thrombotic complication in the late phase.

On the other hand, there have been many researches in which a biodegradable polymer is used as a polymer for carrying therein a biologically active agent. For instance, in Patent Document 3, a medicinal agent such as dexamethasone and a biodegradable polymer such as polylactic acid are dissolved in a solvent, the resulting solution is sprayed onto a stent, and the solvent is evaporated off, to produce a stent which can release the medicinal agent in a sustained manner.

The biodegradable polymer disappears by being decomposed and absorbed into the living body concurrently with or after the release of the biologically active agent. Therefore, the biodegradable polymer has a low risk of causing restenosis or thrombotic complication in the late phase, unlike the non-biodegradable polymers. Among other biodegradable polymers, aliphatic polyesters such as polylactic acid have been widely investigated as polymer for carrying a biologically active agent therein, since they are high in biocompatibility.

However, the aliphatic polyesters such as polylactic acid are hard and brittle, and poor in property for adhesion to a stent. Therefore, the use of an aliphatic ester has a risk of causing cracking or exfoliation of the coating layer during production or transportation of the stent and, further, when the stent is expanded in clinical use thereof. In addition, if the coating layer is breaked, it might be impossible to obtain the desired sustained release of the biologically active agent contained in the coating layer. Besides, if exfoliation of the coating layer is generated during the stent being put indwelling in a coronary artery, the exfoliated fragments may occlude a peripheral blood vessel; this, in the worst case, may induce myocardial infarction.

In addition, where sufficient crimping is not performed in the step of crimping a stent onto a balloon in order to obviate breakage of a coating layer on the stent, the force of gripping of the stent onto the balloon may be lowered, possibly causing slip-off of the stent in a blood vessel during clinical use of the stent.

In order to prevent such exfoliation of the coating layer, a few measures have been proposed. For instance, in Patent Documents 4 and 5, there is mentioned a method in which, for preventing exfoliation of a coating layer from a stent, an intermediate layer is provided between the stent and the coating layer to enhance adhesion therebetween. Patent Document 4 discloses a method in which a coating of parylene is provided as an intermediate layer, and Patent Document 5 discloses a method in which a graft coating of polylactic acid is provided as an intermediate layer. These methods, however, cannot improve the cracking problem of the coating layer, since the methods are not for improving the physical properties of the coating layer. For example, where a strong physical contact occurs during passage of a stent in a guide catheter or during passage of the stent in a hard lesion such as a calcificated lesion, in clinical use of the stent, the possibility of exfoliation of the cracked coating layer cannot be denied.

On the other hand, as a method for improving physical properties of a coating layer to prevent exfoliation of the coating layer, there has been proposed a method of adding a plasticizer to a coating layer so as to enhance flexibility of the coating layer. For instance, as described in Patent Document 6, where glycerin diacetate monocaprylate or diglycerin tetraacetate having a high plasticizing effect to aliphatic polyesters such as polylactic acid is added to a coating layer, cracking of the coating layer can be suppressed.

However, glycerin diacetate monocaprylate and diglycerin tetraacetate have not been used in vivo with favorable results. Therefore, application of such plasticizers to a device to be left implanted in a blood vessel for a long time, such as a stent, has many points unknown about safety. Thus, application of a safer plasticizer is desired.

Besides, as for example shown in Non-patent Document 2, it has been a major matter of concern that a DES newly produced a problem of late stent thrombosis occurring at least one year after the stent was set indwelling in vivo. One of the reasons for this problem is considered to lie in that the medicinal agent carried in the stent not only inhibits the proliferation of smooth muscle cells but also suppresses the formation of a newly formed inner membrane inclusive of endothelial cells. Other than this, as for example shown in Non-patent Document 3, hypersensitivity to the polymer carrying the medicinal agent therein, stent malapposition, and the like are also considered to be major factors leading to the problem.

Thus, the cause of the late stent thrombosis has not yet been elucidated, but attempts to develop and use new DES's for reducing the risk of the thrombosis have already been progressed. For example, Patent Document 7 discloses a stent in which a biodegradable polymer is used as a carrier for a medicinal agent in order to reduce the hypersensitivity to polymer; Patent Document 8 discloses a stent in which no polymer is used and elution of a medicinal agent is controlled by ingenuity of the surface shape of the stent; and Patent Document 9 discloses a stent in which endothelial progenitor cells are captured on the surface of the stent and formation of endothelium is accelerated. On the other hand, Non-patent Document 4 introduces an opinion that the amounts of medicinal agents carried on the existing DES's are more than necessary and that it is desirable to carry a medicinal agent on a stent in a minimum amount necessary and sufficient for suppressing restenosis.

In addition, Patent Document 10 shows an attempt to adopt a different approach aiming at suppression of restenosis by only delivering a medicinal agent through the use of a balloon catheter, without putting any DES indwelling. In this approach, a balloon catheter in which the surface of a balloon is coated with a carcinostatic agent (drug eluting balloon) is used, and the medicinal agent is only caused to migrate to the blood vessel wall at a stenosed lesion speedily at the time of inflation of the balloon. It is said, therefore, that this method is free of a risk of causing the late stent thrombosis, since the balloon does not irritate the blood vessel for a long time, unlike a DES. In fact, Non-patent Document 5 shows very good clinical results with respect to a cure of in-stent restenosis.
Patent Document 1: JP-T-2004-533409
Patent Document 2: JP-T-2004-518458
Patent Document 3: Japanese Patent Laid-open No.
Patent Document 4: JP-T-2001-512354
Patent Document 5: JP-T-2005-516736
Patent Document 6: Japanese Patent Laid-open No. 2007-97706
Patent Document 7: Japanese Patent No. 3673973
Patent Document 8: JP-T-2004-522559
Patent Document 9: JP-T-2003-526477
Patent Document 10: JP-T-2004-524346
Non-patent Document 1: Coronary Intervention, p. 30, Vol. 3, No. 5, 2004
Non-patent Document 2: McFadden EP, Lancet, 2004; 364: 1519-1521
Non-patent Document 3: Joner M, J. Am. Coll. Cardiol., 2006; 48: 193-202
Non-patent Document 4: Aloke VF, Arterioscler Thromb. Vasc. Biol., 2007; 27: 1500-1510
Non-patent Document 5: Scheller B, New Engl. J. Med., 2006 Nov. 16; 355(20): 2113-2124 WO 03/039612 A1 discloses an intraluminal device coated with a therapeutic agent comprised in a matrix. This matrix can be an oil or fat on the basis of triglycerides. Said triglycerides are composed of glycerol and one or more fatty acids, in particular unsaturated fatty acids and more particularly omega-3-fatty-acids. WO 2007/047781 A2 discloses a coating for medical device wherein fat coating comprises a hydrophobic, non-polymeric cross linked gel that contains fatty acids, many of which originate as triglycerides. These triglycerides are further specified to be contained in fish oil and being bonded to omega-3-acids. WO 2006/036967 A1 specifies a coating for a medical device comprising an amount of one or more therapeutic agents dissolved in bioabsorbable carrier component. This bioabsorbable carrier component can comprise among others fatty acid ester or triglycerides. US 2004/0215336 A1 discloses a stent with a drug polymer coating which is composed of various polymeric materials.

### Disclosure of Invention

### Problem to be Solved by the Invention

Accordingly, one of objects of the present invention is to provide an in-vivo indwelling device, for example, a stent which has a low risk of breakage of a coating layer formed on a surface of the stent during production or transportation of the stent and, further, when the stent is expanded during clinical use thereof, and which is high in safety.

Besides, in the prior art, there have been few attempts to reduce the amount of a medicinal agent to be carried on a stent. Further, although it is desirable that also in the case of a drug eluting balloon the amount of a drug for a blood vessel tissue is as small as possible, there have been few attempts to reduce the amount of the medicinal agent carried on the surface of the balloon.

Accordingly, it is another object of the invention to provide an in-vivo indwelling device, for example, a drug delivering medical device with which a decrease in side effects of a remaining medicinal agent can be expected owing to disappearance of the medicinal agent as early as possible, and which can carry thereon the medicinal agent in a minimum amount necessary and sufficient for suppressing restrenosis.

### Means for Solving the Problem

The present inventors have made intensive and extensive investigations in consideration of the above-mentioned circumstances and problems, and, as a result of the investigations, they have completed the present invention. Specifically, the present inventors considered that in the case of adding a plasticizer to a coating layer, not only the effect of the plasticizer to a biodegradable polymer but also the compatibility of materials inclusive of a biologically active agent is important, and they made their earnest investigations. As a result, the present inventors found out that the coating layer can be enhanced in flexibility by adding to the biodegradable polymer a biologically active agent and an additive which has an appropriate degree of lipophilicity.

Furthermore, the present inventors made earnest search for an additive having an appropriate degree of lipophilicity, principally among those drug additives which had been used in vivo, specifically, in blood vessels with good results. As a result, they found additives which are extremely high in safety. Based on the findings, they have completed the present invention.

In addition, it was also found out that addition of a low molecular weight compound having an appropriate degree of lipophilicity together with a medicinal agent causes the medicinal agent to migrate into lumen wall tissues efficiently.

The present invention is achieved by the following (1) to (10).
(1) A stent to be put indwelling in a lumen in vivo, including a a stent main body, and a layer composed of a composition containing a biologically active agent, a biodegradable polymer, and a medium-chain fatty acid triglyceride formed on at least a part of a surface of the stent main body, wherein the medium-chain fatty acid triglyceride is defined by the following chemical formula 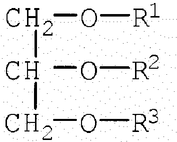 wherein R¹, R², and R³ are each independently a straight-chain acyl group of 6 to 12 carbon atoms, and wherein the addition amount of said medium-chain fatty acid triglyceride in the layer is at least 5 % based on the biodegradable polymer.
(2) The stent according to (1) above, wherein the medium-chain fatty acid triglyceride is a triglyceride synthesized from glycerin and at least one straight-chain saturated fatty acid selected from the group consisting of caprylic acid, capric acid, and a mixture of caprylic acid and capric acid.
(3) The stent according to (1) or (2) above, wherein the medium-chain fatty acid triglyceride is a triglyceride synthesized from glycerin and a straight-chain saturated fatty acid composed of a mixture of caprylic acid and capric acid, and the ratio of the contents of caprylic acid and capric acid in the triglyceride is in the range from 20/80 to 80/20.
(4) The stent according to any of (1) to (3) above, wherein the medium-chain fatty acid triglyceride is contained in the composition in an amount of 5 to 60 wt% based on the biodegradable polymer.
(5) The stent according to any of (1) to (4) above, wherein the biodegradable polymer is at least one polymer selected from the group consisting of polyester, aliphatic polyester, polyacid anhydride, polyorsoester, polycarbonate, polyphosphazene, polyphosphate ester, polyvinyl alcohol, polypeptide, polysaccharide, protein, and cellulose, a copolymer obtained by arbitrary copolymerization of monomers constituting the polymers, or a mixture of the polymer(s) and/or the copolymer(s).
(6) The stent according to any of (1) to (5) above, wherein the biodegradable polymer is an aliphatic polyester.
(7) The stent according to (6) above, wherein the aliphatic polyester is at least one selected from the group consisting of polylactic acid (PLA), polyglycolic acid (PGA), and lactic acid-glycolic acid copolymer (PLGA).
(8) The stent according to any of (1) to (7) above, wherein the biologically active agent is at least one compound selected from the group consisting of carcinostatic agent, immunosuppressor, antibiotic, antirheumatic, antithrombotic agent, HMG-COA reductase inhibitor, ACE inhibitor, calcium antagonist, antilipemia agent, integrin inhibitor, antiallergic agent, antioxidant, GPIIbIIIa antagonist, retinoid, flavonoid, carotenoid, lipid improver, DNA synthesis inhibitor, tyrosine kinase inhibitor, antiplatelet agent, anti-inflammatory agent, bio-derived material, interferon, and NO production accelerating substance.
(9) The stent according to (1) above, which is a drug eluting stent.
(10) The stent according to (1) above, wherein the stent main body is formed from a metal or a polymer.

### Best Mode for Carrying Out the Invention

Now, embodiments of the present invention will be described in detail below.

Incidentally, the present application is based on Japanese Patent Application No. 2007-254540 filed September 28, 2007 and Japanese Patent Application No. 2008-089451 filed March 31, 2008.

By the addition of the medium-chain fatty acid triglyceride, flexibility is imparted to the layer containing the medium-chain fatty acid triglyceride. Consequently, breakage of the layer during production or transportation of the in-vivo indwelling device, for example, a stent and, further, when the stent is expanded during clinical use thereof can be significantly restrained or prevented. In addition, with the breakage of the layer thus restrained or prevented, a desired sustained release can be realized in the case where a medicinal agent or a biologically active agent or the like is contained in the layer. Further, it is possible to restrain such a clinical trouble as occlusion of a peripheral blood vessel by exfoliated fragments of the layer. Furthermore, since the medium-chain fatty acid triglyceride is contained as an accelerator (migration accelerator) for causing the medicinal agent to migrate into lumen wall tissues in vivo, the medicinal agent delivered to the lesion can be made to migrate into the lumen wall tissues efficiently. In other words, the desired suppression of restenosis can be achieved with a smaller amount of the medicinal agent.

The in-vivo indwelling device to be put indwelling in a lumen in vivo includes an in-vivo indwelling device main body, and a layer formed from a composition formed on at least a part of a surface of the in-vivo indwelling device main body, the composition containing a biologically active agent, a biodegradable polymer, and a medium-chain fatty acid triglyceride.

This ensures that in the case where the in-vivo indwelling device main body, for example, the in-vivo indwelling device is applied to a stent, it is possible to control the rate of elution of a biologically active agent from the layer (hereinafter, referred to also as coating layer) composed of a composition containing the biologically active agent, a biodegradable polymer, and a medium-chain fatty acid triglyceride formed on at least a part of a surface of the in-vivo indwelling device main body (for example, stent main body) according to the decomposition (degradation) of the biodegradable polymer. Further, owing to the characteristics of the medium-chain fatty acid triglyceride, a coating layer rich in flexibility can be obtained. Besides, at the same time, it is also possible to restrain or prevent exfoliation of the layer formed on the surface of the stent main body. Furthermore, a crimping operation in mounting the stent onto a balloon can be carried out satisfactorily, and it is possible to prevent such accidents as slip-off of the stent and exfoliation of the coating layer during a curing operation.

In addition, the stent as the in-vivo indwelling device in the present invention is preferably provided with a layer composed of a composition containing a biologically active agent, a biodegradable polymer, and a medium-chain fatty acid triglyceride, on a surface of the stent main body. Since the biodegradable polymer is used to form the layer, as decomposition (degradation) of the biodegradable polymer proceeds in vivo, the biologically active agent is slowly (or sustainedly) released into the living body, which enables appropriate cure. Besides, the medium-chain fatty acid triglyceride in the present invention is a substance the use of which as a drug additive is approved. Further, the medium-chain fatty acid triglycerides include those which are used as a solubilizer in fatty emulsions for phleboclysis and in narcotics, and their metabolic pathway in vivo has been elucidated. Therefore, the medium-chain fatty acid triglycerides can be said to be very high in safety even where they are applied to a device to be implanted in a blood vessel for a long time, such as a stent.

In general, when a medium-chain fatty acid triglyceride is blended into a polymer, the medium-chain fatty acid triglyceride enters among the molecules of the linear polymer, thereby facilitating intermolecular activities. In other words, the medium-chain fatty acid triglyceride is considered to work as so-called "rollers," like balls in ball bearings.

To be more specific, when the medium-chain fatty acid triglyceride such as one that has an appropriate dipole enters into the mutual entanglement of polymer chains, the intermolecular bonds (electromagnetic bonds, van der Waals forces, etc.) inhibiting mutual motions of the polymer chains are weakened, and the mutual entanglement of the polymer chains or the distance between the polymers is loosened or widened. This is considered to result in that flexibility and compatibility are enhanced.

In the layer (hereinafter, referred to also as coating layer) formed from the composition containing a biologically active agent, a biodegradable polymer, and a medium-chain fatty acid triglyceride according to the present invention, the compositional ratio (weight ratio) of the biologically active agent, the biodegradable polymer, and the medium-chain fatty acid triglyceride is preferably in the range from 20:80:48 to 80:20:1, more preferably from 20:80:24 to 80:20:2. This condition is for carrying an appropriate amount of the biologically active agent on the in-vivo indwelling device main body, while taking into account the physical properties and biodegradability of the layer containing the biodegradable polymer.

The addition amount of the medium-chain fatty acid triglyceride in the coating layer according to the present invention is at least 5%, preferably 5 to 60%, more preferably 10 to 30% based on the biodegradable polymer.

If the addition amount ratio is less than 5%, ultra-micro hardness under load is more than 15, and satisfactory flexibility cannot be imparted to the coating layer. On the other hand, if the addition amount ratio is more than 60%, compatibility with the biodegradable polymer is bad, and the medium-chain fatty acid triglyceride is liable to bleed, so that it is difficult to maintain desired physical properties.

Another one of the embodiments of the present invention, preferably, resides in an in-vivo indwelling device in which a composition containing a lipophilic medicinal agent and a medium-chain fatty acid triglyceride as an accelerator for migration of the medicinal agent to tissues is formed on at least a part of a surface brought into contact with a lumen wall tissue in vivo. Specifically, in the in-vivo indwelling device, for example, drug delivering medical device according to the present invention, preferably, a coating layer formed from a composition containing a lipophilic medicinal agent and a medium-chain fatty acid triglyceride is formed on at least a part of a surface brought into contact with a lumen wall tissue in vivo.

This ensures that the medicinal agent is slowly (sustainedly) released from the surface of the in-vivo indwelling device, e.g., drug delivering medical device which is in contact with the lumen wall tissue in vivo into the lumen wall tissue, and the medicinal agent can be efficiently caused to migrate to the lesion. Thus, the in-vivo indwelling device exhibits the effect of a so-called "D.D.S."

The medium-chain fatty acid triglyceride used as the migration accelerator in the present invention is a low molecular weight compound having an appropriate degree of lipophilicity, and is high in affinity for the lipophilic medicinal agent. Therefore, the medicinal agent is present on a surface of the in-vivo indwelling device, e.g., drug delivering medical device in the state of being dissolved or dispersed in the migration accelerator. When the surface of the drug delivering medical device makes contact with the lumen wall tissue in vivo, the medicinal agent migrates into cells of the lumen wall tissue together with the migration accelerator. It is said that, where the medicinal agent is lipophilic, the molecules of the medicinal agent are dissolved in the lipid present at the surfaces of the cell membranes and is moved from the outside into the inside of the cells (active transport). The migration accelerator according to the present invention further accelerates this active transport.

The medium-chain fatty acid triglyceride in the present invention is a substance approved as a drug additive or food additive. Further, the medium-chain fatty acid triglycerides have been used as a solubilizer in fatty emulsions for phleboclysis or in narcotics with good results, and their metabolic pathway in vivo has been elucidated. Therefore, the medium-chain fatty acid triglycerides are particularly effective as very safe additives.

The composition containing the lipophilic medicinal agent and the migration accelerator is present on the whole part or a part of the surface of the stent main body. When the stent is expanded in a stenosed or occluded lesion generated in a lumen, the medicinal agent and the migration accelerator migrate into the cells of the lumen wall tissue. Besides, where sustained (slow) release of the medicinal agent is required, a biodegradable polymer may be made to coexist in the composition for the purpose of controlling the sustained release rate of the medicinal agent. When the sustained drug release stent is expanded in a stenosed or occluded lesion generated in a lumen, the medicinal agent and the migration accelerator are eluted from the polymer, with the lapse of time, to migrate into the cells of the lumen wall tissue.

In the drug delivering medical device of the present invention, preferably the composition containing the lipophilic medicinal agent and the medium-chain fatty acid triglyceride as an accelerator for migration of the medicinal agent to tissues is provided on at least a part of a surface of a hollow expandable body to be brought into contact with a lumen wall tissue in vivo.

This ensures that the lipophilic medicinal agent can be released sustainedly (slowly) into the lumen wall tissue from the surface of the hollow expandable body in contact with the lumen wall tissue in vivo.

As will be described later, the hollow expandable body pertaining to the present invention is the generic name for hollow expandable bodies which have pores or cavities in the inside thereof and which can be contracted and expanded by elasticity or the like. The hollow expandable body is not limited as to whether an end part or parts thereof are closed or open.

The composition containing the lipophilic medicinal agent and the medium-chain fatty acid triglyceride as an accelerator for migration of the medicinal agent to tissues in the present invention further contains a biodegradable polymer.

In the coating layer formed from the composition containing the lipophilic medicinal agent, the medium-chain fatty acid triglyceride, and the biodegradable polymer, the compositional ratio (weight ratio) of the lipophilic medicinal agent, the biodegradable polymer and the medium-chain fatty acid triglyceride is preferably in the range from 20:80:48 to 100:0:1, more preferably from 20:80:24 to 100:0:1, and particularly preferably from 30:70:21 to 100:0:1. This makes it possible to carry the medicinal agent in an appropriate amount, while taking into account the biodegradability of the layer containing the biodegradable polymer.

Besides, as the biodegradable polymer is added to the composition containing the lipophilic medicinal agent and the medium-chain fatty acid triglyceride as an accelerator for migration of the medicinal agent to tissues according to the present invention, the addition amount of the medium-chain fatty acid triglyceride in the present invention is at least 5%, preferably 5 to 60%, more preferably 5 to 30%, based on the biodegradable polymer.

If the addition amount ratio is less than 5%, a sufficient accelerating effect on migration cannot be exhibited. On the other hand, if the addition amount ratio is more than 60%, though a sufficient accelerating effect on migration can be obtained, an effect by which the medicinal agent having migrated into the cells are pushed out of the cells is exhibited more strongly than the accelerating effect, so that it becomes impossible to maintain an effective medicinal agent concentration in the cells.

Now, each of the essential components of the in-vivo indwelling device according to the present invention will be described in detail below.

### "Medium-chain fatty acid triglyceride"

The medium-chain fatty acid triglyceride in the present invention is a triglyceride synthesized from a straight-chain saturated fatty acid of 6 to 12 carbon atoms and glycerin, preferably a triglyceride synthesized from a straight-chain saturated fatty acid of 6 to 10 carbon atoms and glycerin, and more preferably a triglyceride synthesized from a straight-chain saturated fatty acid of 8 to 10 carbon atoms and glycerin.

In the case of a triglyceride synthesized from a straight-chain saturated fatty acid of less than 6 carbon atoms and glycerin, the triglyceride is so low in lipophilicity that it shows poor compatibility with the lipophilic medicinal agent or biologically active agent and the optionally added biodegradable polymer. Consequently, flexibility of the coating layer cannot be enhanced and/or effective migration of the medicinal agent into cells cannot be achieved. On the other hand, in the case of a triglyceride synthesized from a straight-chain saturated fatty acid of more than 12 carbon atoms and glycerin, the triglyceride is so high in crystallinity that its dispersibility to the medicinal agent or biologically active agent and the biodegradable polymer is bad, and effective migration of the medicinal agent into cells cannot be achieved. In contrast, the triglyceride synthesized from a straight-chain saturated fatty acid of 6 to 12 carbon atoms and glycerin has an appropriate degree of lipophilicity, so that it is high in compatibility with the medicinal agent or biologically active agent and the biodegradable polymer. Consequently, flexibility of the coating layer can be enhanced, and effective migration of the medicinal agent into cells can be achieved.

When the medium-chain fatty acid triglyceride in the present invention is added to the coating layer (the layer formed on at least a part of a surface of a stent main body and formed from the composition containing the biologically active agent, the biodegradable polymer, and the medium-chain fatty acid triglyceride) formed on a surface of the in-vivo indwelling device in the present invention, i.e. on a surface of a stent, flexibility can be imparted to the coating layer. In addition, as a method for quantitatively measuring the flexibility of a very thin layer such as the above-mentioned coating layer, measurement of ultra-micro hardness under load (also called "HTL," "dynamic hardness" and "universal hardness") by use of an ultra-micro hardness tester is used preferably. In this method, as described in JIS Z2255, hardness of a solid material is measured as a resistance to a triangular pyramid diamond indenter when the indenter is pressed against the surface of a specimen, and it is possible by the method to measure hardness in a very minute region of a solid material having a thickness of not less than 10 µm. In order that such a damage as cracking of the coating layer or exfoliation of the coating layer from the stent main body is not generated when the stent is expanded, the ultra-micro hardness under load is desirably 15 or below, more preferably 12 or below, and particularly preferably 10 or below. The present inventors have made earnest search for a substance which causes the ultra-micro hardness under load of the coating layer to be 15 or below and which is high in safety, among a large number of additives. As a result of the search, they found the medium-chain fatty acid triglycerides according to the present invention.

The medium-chain fatty acid triglycerides according to the present invention may be one synthesized from the straight-chain saturated fatty acid of the above-mentioned number of carbon atoms and glycerin, or may be a commercially available one, and is not particularly limited. Where the medium-chain fatty acid triglyceride according to the present invention is synthesized and put to use, the synthesis can be performed in the same conditions as those for known methods such as dehydration condensation. In either of the case where a commercialized one is purchased and the case where a newly synthesized one is used, the medium-chain fatty acid triglyceride according to the present invention is preferably one that satisfies the following chemical formula 1: 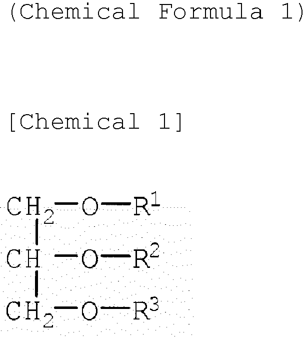 (in the chemical formula 1, R¹, R², and R³ are each independently a straight-chain acyl group of 6 to 12 carbon atoms).

In addition, the straight-chain acyl group of 6 to 12 carbon atoms is specifically an acyl group in which the carbon atom of a carbonyl group is bonded to a carbon atom at the 1-position constituting the terminal end of at least one group selected from the group consisting of hexyl group, heptyl group, octyl group, nonyl group, decyl group, undecyl group, and dodecyl group. Besides, the groups R¹, R², and R³ in the above formula may all be the same or different.

The medium-chain fatty acid triglyceride in the present invention is preferably a triglyceride synthesized from glycerin and at least one straight-chain saturated fatty acid selected from the group consisting of caproic acid, caprylic acid, capric acid, lauric acid, and a mixture of caprylic acid and capric acid. Specific examples of this triglyceride, referring to the above chemical formula 1, include a triglyceride (tricaproin) in which R¹, R², and R³ are CH₃(CH₂)₄-(CO)-; a triglyceride (tricaprylin) in which R¹, R², and R³ are CH₃(CH₂)₆-(CO)-; a triglyceride (tricaprin) in which R¹, R², and R³ are CH₃(CH₂)₈-(CO)-; a triglyceride in which R¹ and R² are CH₃(CH₂)₆-(CO)- and R³ is CH₃(CH₂)₈-(CO)-; a triglyceride in which R¹ and R² are CH₃(CH₂)₈-(CO)- and R³ is CH₃(CH₂)₆-(CO)-; a triglyceride in which R¹ and R³ are CH₃(CH₂)₆-(CO)- and R² is CH³(CH₂)₈-(CO)-; a triglyceride in which R¹ and R³ are CH₃(CH₂)₈-(CO)- and R² is CH₃(CH₂)₆-(CO)-; a triglyceride (trilaurin) in which R¹, R², and R³ are all CH₃(CH₂)₁₀-(CO)-; and mixtures of these triglycerides.

More preferably, the medium-chain fatty acid triglyceride in the present invention is a triglyceride synthesized from glycerin and at least one straight-chain saturated fatty acid selected from the group consisting of caprylic acid, capric acid, and a mixture of caprylic acid and capric acid. Specific examples of this triglyceride, referring to the above chemical formula 1, include a triglyceride (tricaprylin) in which R¹, R², and R³ are CH₃(CH₂)₆-(CO)-; a triglyceride (tricaprin) in which R¹, R², and R³ are CH₃(CH₂)₈-(CO)-; a triglyceride in which R¹ and R² are CH₃(CH₂)₆-(CO)- and R³ is CH₃(CH₂)₈-(CO)-; a triglyceride in which R¹ and R² are CH₃(CH₂)₈-(CO)- and R³ is CH₃(CH₂)₆-(CO)-; a triglyceride in which R¹ and R³ are CH₃(CH₂)₆-(CO)- and R² is CH₃(CH₂)₈-(CO)-; a triglyceride in which R¹ and R³ are CH₃(CH₂)₈-(CO)- and R² is CH₃(CH₂)₆-(CO)-; and mixtures of these triglycerides.

Particularly preferably, the medium-chain fatty acid triglyceride in the present invention is a triglyceride synthesized from glycerin and a straight-chain saturated fatty acid composed of a mixture of caprylic acid and capric acid, and is composed of a mixture of the above-mentioned six triglycerides. Examples of triglycerides commercialized at present as medium-chain fatty acid triglyceride composed of a mixture of the six triglycerides include Miglyol 810, and Miglyol 812.

Where the medium-chain fatty acid triglyceride in the present invention is a triglyceride synthesized from glycerin and a straight-chain saturated fatty acid composed of a mixture of caprylic acid and capric acid, the content ratio by weight of caprylic acid and capric acid is preferably in the range from 20/80 to 80/20; more preferably, the content ratio of the caprylic acid and capric acid in one molecule of the triglyceride is in the range from 50/50 to 80/20.

Where the content ratio by weight is in such a range, the ultra-micro hardness under load of the coating layer can be made to be 15 or below, and satisfactory flexibility can be imparted to the coating layer. Further, where the number of carbon atoms is in the above-mentioned range and the content ratio of the caprylic acid and capric acid is in the above-mentioned range, the triglyceride has an appropriate degree of lipophilicity, so that it is high in compatibility with the biodegradable polymer and the lipophilic medicinal agent, and effective migration of the medicinal agent into cells can be achieved.

Incidentally, the expression "the content ratio by weight of caprylic acid and capric acid" herein means the weight ratio (mass ratio) of caprylic acid and capric acid which are obtained upon decomposition of the medium-chain fatty acid triglyceride in the present invention to glycerin and the straight-chain saturated fatty acids.

### "Biodegradable polymer"

The biodegradable polymer in the present invention is not particularly limited insofar as it is a polymer which is slowly biodegraded when the in-vivo indwelling device according to the present invention is put indwelling in a lesion and which does not exert bad influence on humans or animals. Preferably, the biodegradable polymer is, for example, at least one polymer selected from the group consisting of aliphatic polyester, polyester, polyacid anhydride, polyorsoester, polycarbonate, polyphosphazene, polyphosphate ester, polyvinyl alcohol, polypeptide, polysaccharide, protein, and cellulose, a copolymer obtained by arbitrary copolymerization of monomers constituting the polymers, or a mixture of the polymer(s) and/or the copolymer(s). Incidentally, the term "mixture" herein refers to a wide concept inclusive of composites such as polymer alloys. Among these, aliphatic polyesters are preferred, since they are low in reactivity with living tissues and their decomposition (degradation) in vivo can be controlled.

The aliphatic polyester is not particularly limited. Preferably, the aliphatic polyester is, for example, at least one polymer selected from the group consisting of polylactic acid, polyglycolic acid, polyhydroxybutyric acid, polyhydroxyvaleric acid, polyhydroxypentanoic acid, polyhydroxyhexanoic acid, polyhydroxyheptanoic acid, polycaprolactone, polytrimethylene carbonate, polydioxane, polymalic acid, polyethylene adipate, polyethylene succinate, polybutylene adipate, and polybutylene succinate, a copolymer obtained by arbitrary copolymerization of monomers constituting the polymers, or a mixture of the polymer(s) and/or the copolymer(s). Among these, more preferable are polylactic acid (PLA), polyglycolic acid (PGA), and lactic acid-glycolic acid copolymer (PLGA), since they are high in biocompatibility and their decomposition (degradation) in vivo can be easily controlled.

The polylactic acid (PLA), polyglycolic acid (PGA), and lactic acid-glycolic acid copolymer (PLGA) can be obtained by dehydration polycondensation from one or more compounds selected from among L-lactic acid, D-lactic acid and glycolic acid paying attention to the molecular structure. Preferably, the PLA, PGA, and PLGA can be obtained by ring-opening polymerization from one or more compounds selected, according to the necessary structure, from among lactides, which are each a cyclic dimers of lactic acid, and glycolides, which are each a cyclic dimer of glycolic acid. The lactides include L-lactide, which is a cyclic dimer of L-lactic acid, D-lactide, which is a cyclic dimer of D-lactic acid, mesolactide, which is a cyclic dimerization product of D-lactic acid and L-lactic acid, and DL-lactide, which is a racemic mixture of D-lactide and L-lactide. In the present invention, any of the above-mentioned lactides can be used.

In addition, the weight average molecular weight of the biodegradable polymer in the present invention is preferably in the range 10,000 to 1,000,000, more preferably 20,000 to 500,000, and particularly preferably 50,000 to 200,000. Incidentally, examples of the method for measuring weight average molecular weight include GPC, light-scattering method, viscometry, and mass spectrometry (TOFMASS, etc.). For the biodegradable polymer in the present invention, measurement of weight average molecular weight is preferably carried out by GPC.

### "Medicinal agent"

The lipophilic medicinal agent in the present invention is for suppressing stenosis or occlusion which might generated in a lumen system when the in-vivo indwelling device according to the present invention is put indwelling in a lesion. The lipophilic medicinal agent is not particularly limited, and can be selected arbitrarily, insofar as it is a lipophilic one. Specifically, at least one selected from the group consisting of carcinostatic agent, immunosuppressor, antibiotic, antithrombotic agent, HMG-CoA reductase inhibitor, ACE inhibitor, calcium antagonist, antilipemia agent, integrin inhibitor, antiallergic agent, antioxidant, GPIIbIIIa antagonist, retinoid, lipid improver, antiplatelet agent, and anti-inflammatory agent may be used as the lipophilic medicinal agent, since it can control the behavior of cells in the tissue of a lesion and can cure the lesion.

Preferable examples of the carcinostatic agent include paclitaxel, docetaxel, vinblastin, vindesin, irinotecan, pirarubicin, and etc.

Preferable examples of the immunosuppressor include sirolimus, sirolimus derivative such as everolimus, pimecrolimus, ABT-578 and the like, tacrolimus, azathioprine, cyclosporine, cyclophosphamide, mycophenolate mofetil, gusperimus, and etc.

Preferable examples of the antibiotic include mitomycin, adriamycin, doxorubicin, actinomycin, daunorubicin, idarubicin, pirarubicin, aclarubicin, epirubicin, zinostatin stimalamer, and etc.

Preferable examples of the antithrombotic agent include aspirin, ticlopidine, and etc.

Preferable examples of the HMG-CoA reductase inhibitor include cerivastatin, cerivastatin sodium, atorvastatin, pitavastatin, fluvastatin, fluvastatin sodium, simvastatin, lovastatin, and etc.

Preferable examples of the ACE inhibitor include quinapril, trandolapril, temocapril, delapril, enalapril maleate, captopril, and etc.

Preferable examples of the calcium antagonist include hifedipine, nilvadipine, benidipine, nisoldipine, and etc.

Preferable examples of the antilipemia agent include probucol.

Preferable examples of the integrin inhibitor include AJM300.

Preferable examples of the antiallergic agent include tranilast.

Preferable examples of the antioxidant include α-tocopherol, catechin, dibutylhydroxytoluene, and butylhydroxyanisole.

Preferable examples of the GPIIbIIIa antagonist include abciximab.

Preferable examples of the retinoid include all-trans-retinoic acid.

Preferable examples of the lipid improver include eicosapentaenoic acid.

Preferable examples of the antiplatelet agent include ticlopidine, cilostazol (500), and clopidogrel.

Preferable examples of the anti-inflammatory agent include steroids such as dexamethasone, prednisolone and the like.

The lipophilic medicinal agent in the present invention preferably has an oil/water distribution coefficient of not less than 1, more preferably in the range from 5 to 100,000,000, and further preferably in the range from 10 to 50,000,000.

If the oil/water distribution coefficient of the lipophilic medicinal agent in the present invention is less than 1, compatibility of the medicinal agent with the medium-chain fatty acid triglyceride is poor. If the oil/water distribution coefficient exceeds 100,000,000, on the other hand, the medicinal agent may be incapable of being dissolved or dispersed in the medium-chain fatty acid triglyceride.

The term "oil/water distribution coefficient" herein is n-octanol/water distribution coefficient, which is a numerical value indicating whether the proportion of a portion dissolved in oil is higher or the proportion of a portion dissolved in water is higher. Medicinal agents having an oil/water distribution coefficient of more than 1 are classified as lipophilic medicinal agents, and medicinal agents having an oil/water distribution coefficient of less than 1 are classified as watersoluble medicinal agents.

The coating layer in the present invention is composed of a composition containing the lipophilic medicinal agent, the medium-chain fatty acid triglyceride, and the biodegradable polymer (hereinafter the layer composed of the composition containing the lipophilic medicinal agent, the medium-chain fatty acid triglyceride, and the biodegradable polymer will be referred to also as covering layer).

The covering layer in the present invention may contain only one of the above-mentioned medicinal agents, or may contain two or more different ones of the above-mentioned medicinal agents. Where two or more medicinal agents are contained in the coating layer, the combination of the medicinal agents may be appropriately selected from the above-mentioned medicinal agents, as required. It is to be noted here, however, that the medicinal agent in the present invention is preferably an immunosuppressor or a carcinostatic agent, and more preferably an immunosuppressor such as sirolimus, sirolimus derivative such as everolimus, pimecrolimus, ABT-578, AP23573, CCI-779 and the like, tacrolimus, etc. or a carcinostatic agent such as paclitaxel, docetaxel, etc.

This ensures that by inserting the in-vivo indwelling device according to the present invention, for example, a drug delivering medical device having a hollow expandable body into an artery or a vein serving as an example of a lumen wall tissue in vivo, an EPR (Enhanced Permeability and Retention effect of Macromolecules and lipids) effect by the composition containing the medium-chain fatty acid triglyceride and the lipophilic medicinal agent is considered to be expectable.

Besides, in the case where the covering layer in the present invention further contains the biodegradable polymer, the compositional ratio (weight ratio) of the lipophilic medicinal agent and the biodegradable polymer in the coating layer in the present invention is preferably in the range from 1:99 to 99:1, more preferably in the range from 20:80 to 80:20. This is for carrying an appropriate amount of the medicinal agent, while taking into account the physical properties of the covering layer and the sustained release property of the medicinal agent.

### "Biologically active agent"

The biologically active agent in the present invention is not particularly limited, and can be selected arbitrarily, insofar as it suppresses stenosis or occlusion which might occur in a lumen system when the in-vivo indwelling device, for example a stent, according to the present invention is put indwelling in a lesion. The use of at least one selected from the group consisting of carcinostatic agent, immunosuppressor, antibiotic, antirheumatic, antithrombotic agent, HMG-COA reductase inhibitor, ACE inhibitor, calcium antagonist, antilipemia agent, integrin inhibitor, antiallergic agent, antioxidant, GPIIbIIIa antagonist, retinoid, flavonoid, carotenoid, lipid improver, DNA synthesis inhibitor, tyrosine kinase inhibitor, antiplatelet agent, anti-inflammatory agent, bio-derived material, interferon, and NO production accelerating substance, as the biologically active agent is preferable, since it is thereby possible to control the behavior of cells in the tissue of a lesion and to cure the lesion.

Preferable examples of the carcinostatic agent include vincristin, vinblastin, vindesin, irinotecan, pirarubicin, paclitaxel, docetaxel, methotrexate, and etc.

Preferable examples of the immunosuppressor include sirolimus, sirolimus derivative such as everolimus, pimecrolimus, ABT-578, AP23573, CCI-779 and the like, tacrolimus, azathioprine, cyclosporine, cyclophosphamide, mycophenolate mofetil, gusperimus, mizoribin, and etc, of which more preferable are sirolimus, sirolimus derivative such as everolimus, pimecrolimus, ABT-578, AP23573, CCI-779 and the like, and tacrolimus.

Preferable examples of the antibiotic include mitomycin, adriamycin, doxorubicin, actinomycin, daunorubicin, idarubicin, pirarubicin, aclarubicin, epirubicin, peplomycin, zinostatin stimalamer, and etc.

Preferable examples of the antirheumatic include methotrexate, sodium thiomalate, penicillamine, lobenzarit, and etc.

Preferable examples of the antithrombotic agent include heparin, aspirin, antithrombin preparation, ticlopidine, hirudin, and etc.

Preferable examples of the HMG-COA reductase inhibitor include cerivastatin, cerivastatin sodium, atorvastatin, rosuvastatin, pitavastatin, fluvastatin, fluvastatin sodium, simvastatin, lovastatin, pravastatin, and etc.

Preferable examples of the ACE inhibitor include quinapril, perndopril erbumine, trandolapril, cilazapril, temocapril, delapril, enalapril maleate, lisinopril, captopril, and etc. Preferable examples of the calcium antagonist include hifedipine, nilvadipine, diltiazem, benidipine, nisoldipine, and etc.

Preferable examples of the antilipemia agent include probucol. Preferable examples of the integrin inhibitor include AJM300. Preferable examples of the antiallergic agent include tranilast. Preferable examples of the antioxidant include α-tocopherol. Preferable examples of the GPIIbIIIa antagonist include abciximab.

Preferable examples of the retinoid include all-trans-retinoic acid. Preferable examples of the flavonoid include epigallocatechin, anthocyanine, and proanthocyanidin. Preferable examples of the carotinoid include β-carotene, and lycopene. Preferable examples of the lipid improver include eicosapentaenoic acid.

Preferable examples of the DNA synthesis inhibitor include 5-FU. Preferable examples of the tyrosine kinase inhibitor include genistein, tyrphostin, erbstatin, staurosporine, and etc. Preferable examples of the antiplatelet agent include ticlopidine, cilostazol, clopidogrel, and etc. Preferable examples of the anti-inflammatory agent include steroids such as dexamethasone, prednisolone and the like.

Preferable examples of the bio-derived material include EGF (epidermal growth factor), VEGF (vascular endothelial growth factor), HGF (hepatocyte growth factor), PDGF (platelet derived growth factor), BFGF (basic fibrolast growth factor), and etc.

Preferable examples of the interferon include interferon-γ1a. Preferable examples of the NO production accelerating substance include L-alginine.

The coating layer in the present invention may contain only one of the above-mentioned biologically active agents, or may contain two or more different ones of the above-mentioned biologically active agents. Where two or more biologically active agents are contained in the coating layer, the combination of the biologically active agents may be appropriately selected from the above-mentioned biologically active agents, as required. It is to be noted here, however, that the biologically active agent in the present invention is preferably sirolimus, sirolimus derivative such as everolimus, pimecrolimus, ABT-578, AP23573, CCI-779 or the like, or tacrolimus, of which sirolimus or sirolimus derivative is more preferable.

Besides, in the coating layer in the present invention, the compositional ratio (weight ratio) of the biologically active agent and the biodegradable polymer is preferably in the range from 1:99 to 99:1, more preferably from 20:80 to 80:20. This is for carrying an appropriate amount of the biologically active agent, while taking into account the physical properties of the coating layer and sustained release property of the biologically active agent.

### "Hollow expandable body"

The hollow expandable body, i.e. the stent pertaining to the present invention is a medical device which has a hollow structure and is expanded (or inflated) when inserted in a predetermined position in a living body. The shape of such hollow expandable body is preferably a cylindrical shape, a tubular shape, or a polygonal or substantially spherical shape having a cavity or cavities therein, and the hollow expandable body is preferably annular in section.

Now, a stent having a stent main body or a hollow expandable body will be described in detail below as the in-vivo indwelling device according to the present invention. Naturally, the stent described below is not limitative of the drug delivering medical device having a hollow expandable body pertaining to the present invention.

"Stent"

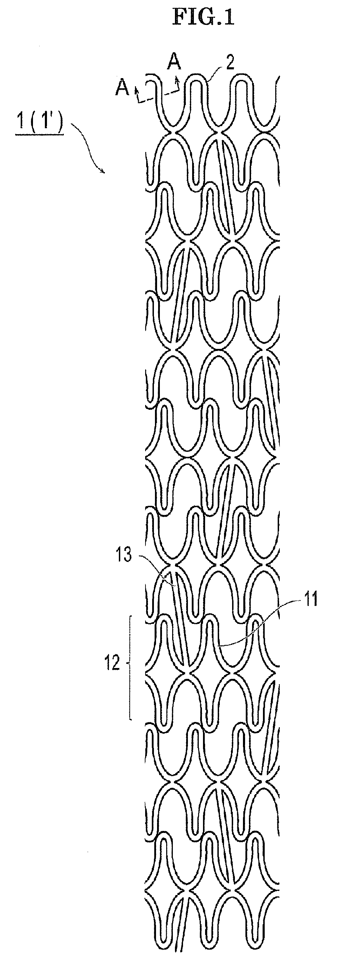

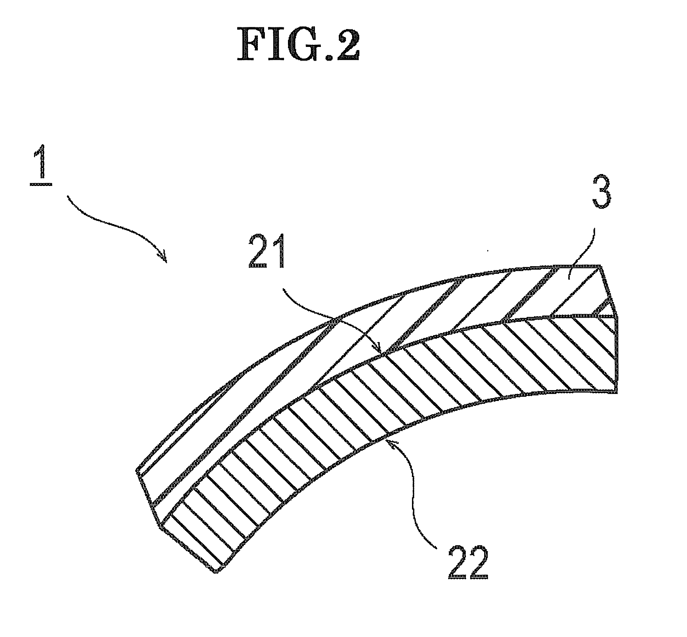

FIG. 1 is a side view showing an embodiment of a stent as the in-vivo indwelling device according to the present invention, and FIG. 2 is an enlarged cross-sectional view taken along line A-A of FIG. 1. Now, each of essential components of the stent 1 will be described in detail below.

The stent 1 (main body 1') is a hollow cylindrical body which opens at both terminal ends thereof and which extends in the longitudinal direction between both terminal ends thereof. A side surface of the hollow cylindrical body is provided with a multiplicity of cutouts offering communication between the outside surface and the inside surface of the hollow cylindrical body. With the cutouts deformed, the hollow cylindrical body can be expanded and contracted in the radial direction thereof. The stent 1 is put indwelling in a body lumen such as vessel (e.g., blood vessel) or bile duct, and maintains its shape.

In the embodiment shown in FIG. 1, the stent main body has, as a basic unit thereof, a substantially rhombic element 11 composed of elastic linear material and having a cutout therein. A plurality of the substantially rhombic elements 11 are linked so that the substantially rhombic shapes are continuously arrayed in their minor axis direction, to form an annular unit 12. The annular unit 12 is connected to the adjacent annular unit through a linear elastic member 13. This ensures that the plurality of annular units 12 are arranged continuously in their axial direction, in the state of being mutually linked in part. With such a configuration, the stent (main body) forms a hollow cylindrical body which opens at both terminal ends thereof and extends in the longitudinal direction between both terminal ends thereof. Besides, the side surface of the hollow cylindrical body has the substantially rhombic cutouts, and, with the cutouts deformed, the hollow cylindrical body can be expanded and contracted in the radial direction thereof.

It should be noted here, however, that the structure of the stent as an example of the in-vivo indwelling device according to the present invention is not limited to the embodiment shown in the figures. Specifically, the stent structure refers to a concept widely including structures of hollow cylindrical bodies which are open at both terminal ends thereof, extend in the longitudinal direction between both terminal ends thereof, have a side surface provided with a multiplicity of cutouts offering communication between the outside surface and the inside surface thereof, and which can be expanded and contracted in the radial direction thereof through deformation of the cutouts. Further, the stent structure according to the present invention means a concept including coiled form structure. The sectional shape of the elastic linear material constituting the stent (main body), also, is not limited to a rectangle as shown in FIG. 2, and may be other shape such as circle, ellipse, and polygons other than rectangle.

In the stent 1 as an example of the in-vivo indwelling device according to the present invention, as shown in FIG. 2, on the surface of a linear member 2 constituting the stent main body 1', a layer 3 (coating layer or covering layer) formed from a composition containing a lipophilic medicinal agent or biologically active agent and a medium-chain fatty acid triglyceride and (optionally) a biodegradable polymer is formed so as to cover at least a part of the linear member 2. Incidentally, the layer 3 may cover the surface of the stent main body 1' either entirely or partly, or may be formed only on the outside surface 21 of the linear member 2 as shown in FIG. 2. FIG. 2 only shows a schematic drawing in which a part of the surface of the linear member 2 constituting the stent main body 1' is covered.

Further, the layer 3 is preferably formed on at least that part of the linear member 2 which is brought into direct contact with a living tissue. This ensures that the lipophilic medicinal agent or biologically active agent released from the layer can be absorbed directly through the living tissue without flowing in a body fluid, for example, blood. Therefore, local administration of the medicinal agent can be performed, and a more effective pharmacological activity can be achieved.

Incidentally, while FIGS. 1 and 2 show an example of the section of the stent in the case where the stent main body 1' is made of metal, the present invention is not limited to the cases where the stent main body 1' is made of metal.

Incidentally, as the in-vivo indwelling device or the drug delivering medical device having a hollow expandable body according to the present invention is a stent, the in-vivo indwelling device main body and the hollow expandable body correspond to the stent main body 1'. Besides, the device in which the layer formed from the composition containing the biologically active agent or lipophilic medicinal agent and the medium-chain fatty acid triglyceride and the biodegradable polymer is coveringly formed on at least a part of the surface of the stent main body 1' corresponds to the stent, that is, the in-vivo indwelling device or the drug delivering medical device having a hollow expandable body according to the present invention.

Now, the stent main body pertaining to the present invention will be described below.

### "Stent main body"

Examples of the material for the stent main body pertaining to the present invention include polymer materials, metallic materials, carbon fiber, ceramics, and the like, which may be used either singly or in appropriate combination. The material is not particularly limited, insofar as it has certain degrees of rigidity and elasticity. The material is preferably a biocompatible material, more preferably a metal, a polymer material or carbon fiber, and further preferably a metal or a polymer material.

Specific and preferable examples of the polymer material include polyolefins such as polyethylene, polypropylene, etc., aromatic polyesters such as polyethylene terephthalate, etc., aliphatic polyesters such as polylactic acid, polyglycolic acid, etc., cellulose-based polymers such as cellulose acetate, cellulose nitrate, etc., and fluorine-containing polymers such as polytetrafluoroethylene, tetrafluoroethylene-ethylene copolymer, etc.

Preferable examples of the metallic material include stainless steels, tantalum, tantalum alloys, titanium, titanium alloys, nickel-titanium alloys, tantalum-titanium alloys, nickel-aluminum alloys, inconel, gold, platinum, iridium, tungsten, tungsten alloys, cobalt-based alloys, and the like. Among the stainless steels, preferred is SUS316L, which is the highest in corrosion resistance. Among the cobalt-based alloys, preferred are MP35N, L605, etc. Among the tungsten alloys, preferred are W-Rh25% and W-Rh26%.

The stent main body in the present invention can be formed favorably from a material which is appropriately selected from the above-mentioned materials according to the application site or expanding means. For instance, where the stent main body is formed from a metallic material, the excellent strength of the metallic material enables the stent to be assuredly set indwelling in a lesion. Where the stent main body is formed from a polymer material, the excellent flexibility of the polymer material promises excellent deliverability of the stent to a lesion.

In addition, where the stent main body is formed from a biodegradable polymer such as polylactic acid, the stent itself disappears by being decomposed (degraded) and absorbed into the living body after its function as a stent is displayed, in other words, after acute-stage blood vessel occulusion and restenosis rate are suppressed. Therefore, this type of stent shows an excellent effect in that there is a low risk of causing late-phase restenosis or thrombotic complication.

Besides, where the stent is of the self-expandable type, a restoring force for returning to an original shape is required and, therefore, a superelastic alloy (e.g., nickel-titanium alloy) and the like is preferable as the material. Where the stent is of the balloon-expandable type, it is desirable that return to a former shape after expansion would not easily occur, and, therefore, stainless steel and the like is preferable as the material.

In addition, where the stent main body is formed from carbon fiber, the high strength and excellent flexibility of the carbon fiber display an excellent effect in that safety of the stent in vivo is high.

The size of the stent main body in the present invention may be appropriately selected according to the application site. For example, where the stent is to be used for a coronary artery of a heart, normally, the outside diameter of the stent main body before expanded is preferably 1.0 to 3.0 mm, and the length is preferably 5 to 50 mm. As shown in FIG. 1, where the stent main body is composed of a linear member, the length in width direction of the linear member constituting the stent main body so as to have a multiplicity of cutouts is preferably 0.01 to 0.5 mm, more preferably 0.05 to 0.2 mm.

The method for producing the stent main body in the present invention is not particularly limited, and may be appropriately selected from normally used production methods, according to the structure and material(s) of the stent. Examples of the production methods which can be selected here include those utilizing an etching technology such as laser etching, chemical etching, etc. and those utilizing a laser cutting technology.

The thickness of the coating layer or covering layer 3 in the present invention is set within a range such that the performances of the stent main body such as deliverability to a lesion and stimulus to blood vessel walls are not considerably spoiled and that the effect of the release of the lipophilic medicinal agent is exhibited sufficiently. The average thickness of the layer 3 is preferably 1 to 75 µm, more preferably 1 to 30 µm, and further preferably 1 to 10 µm. Where the average thickness of the layer 3 is in the range of 1 to 75 µm, an excellent effect for sustained released of the lipophilic medicinal agent or biologically active agent when the stent is put indwelling in a living lumen is promised, the stent 1 itself is not too large in outside diameter, there is a low risk of causing a trouble in delivering the stent to a lesion, the stent does not irritate the blood vessel walls, and restenosis can be suppressed or prevented.

The covering layer in the present invention is composed of the composition containing the lipophilic medicinal agent, the medium-chain fatty acid triglyceride, and optionally the biodegradable polymer. Besides, the coating layer in the present invention is composed of the composition containing the biologically active agent, the biodegradable polymer, and the medium-chain fatty acid triglyceride. It is to be noted here, however, that the covering layer and the coating layer may not necessarily cover the whole surface of the linear member 2 constituting the stent main body, and must only cover at least a part of the surface of the linear member 2 constituting the stent main body. Therefore, a structure may be adopted in which only the upper side of the outside surface 21 of the cross sectional shape as shown in FIG. 2 (the inside surface 22 is forming a shorter arc, and the outside surface 21 is forming a relatively longer arc) is covered (coated) with the layer 3. In the case of the layer 3 formed in this manner, the lipophilic medicinal agent or biologically active agent is locally released from the stent surface into the living tissue, so that an effective cure can be performed. In addition, the drug delivering medical device having a hollow expandable body as an example of the in-vivo indwelling device according to the present invention is preferably a drug eluting stent.

Besides, the covering layer and the coating layer in the present invention preferably cover 1 to 80% of the whole surface area of the stent main body, more preferably 10 to 50% of the whole surface area of the stent main body.

Now, an embodiment of the method of producing the stent as an example of the in-vivo indwelling device according to the present invention will be described below, but the invention is not to be limited to the embodiment.

### "Preparation of composition containing biologically active agent, biodegradable polymer, and medium-chain fatty acid triglyceride"

The biologically active agent, the biodegradable polymer and the medium-chain fatty acid triglyceride are dissolved in a solvent such as acetone, ethanol, chloroform, tetrahydrofuran, etc. in the above-mentioned ratio so that the solution concentration (the total concentration of the biologically active agent, the biodegradable polymer and the medium-chain fatty acid triglyceride in the solvent) is in the range of 0.001 to 20 wt%, preferably 0.01 to 10 wt%, to prepare a composition containing the biologically active agent, the biodegradable polymer, and the medium-chain fatty acid triglyceride pertaining to the present invention.

### "Preparation of composition containing lipophilic medicinal agent, medium-chain fatty acid triglyceride, and biodegradable polymer"

The lipophilic medicinal agent, the medium-chain fatty acid triglyceride, and optionally the biodegradable polymer are dissolved in a solvent such as acetone, ethanol, chloroform, tetrahydrofuran, etc. in the above-mentioned ratio, or in a predetermined mixing ratio, so that the solution concentration (the total concentration of the medicinal agent, the biodegradable polymer and the medium-chain fatty acid triglyceride in the solvent) is 0.001 to 20 wt%, preferably 0.01 to 10 wt%, to prepare a composition containing the medicinal agent, the biodegradable polymer, and the medium-chain fatty acid triglyceride pertaining to the present invention.

### "Method for producing stent main body"

The method for producing the stent main body in the present invention is not particularly limited, and the stent main body can be produced by use of a known method.

In the case where the stent main body in the present invention is formed from a metallic material, as for example described in Japanese Patent Laid-open No. 2007-144108, a metal (inclusive of alloy) such as Ni and Ti is cold worked to produce a superelastic metal pipe. The metallic pipe is set on a rotary motor equipped with a fastener mechanism for preventing offset of the axis, and, further, the assembly is set on an X-table capable of numerical control. Thereafter, the X-table and the rotary motor are connected to a personal computer, and, with outputs from the personal computer inputted to an numerical controller for the X-table and to the rotary motor, the X-table and the rotary motor are driven based on drawing data outputted from the personal computer. The metallic pipe thus set is irradiated with a laser beam, whereby a stent main body having a predetermined shape can be produced. In the case of, for example, a stent having a coiled form, the metal pertaining to the present invention is formed into a wire having a predetermined thickness (diametral size) and a predetermined outer shape. Then, the wire is bent into a pattern such as a wavy pattern, followed by spirally winding the wire around a mandrel. Thereafter, the mandrel is pulled out, and the thus shaped product of wire is cut to a predetermined length, whereby a coiled form stent main body can be produced. Other examples of the stent main body include: a stent main body based on an example disclosed in Japanese Patent Laid-open No. Hei 9-215753 and Japanese Patent Laid-open No. Hei 7-529, wherein an elastic linear material is bent into a coil form and a plurality of such coils are connected to form a hollow cylindrical body, and wherein the gaps between portions of the elastic wire material constitute cutouts; and a stent main body based on an example disclosed in JP-T-H8-502428 and JP-T-H7-500272, wherein an elastic linear material is bent into a zigzag form and a plurality of the zigzag members are connected to form a hollow cylindrical body, and wherein the gaps between portions of the elastic linear material constitute cutouts.

Besides, in the case where the stent main body in the present invention is formed from a polymer material, the polymer material is molded into a pipe having a predetermined material thickness and a predetermined outer shape by use of an injection molding machine, an extruder, a press molding machine, a vacuum forming machine, a blow molding machine, and the like. Then, an aperture pattern is adhered to the surface of the pipe, and the pipe portions other than the aperture pattern are melted away by an etching technique such as laser etching and chemical etching, to form apertures. Or, alternatively, apertures can also be formed by a method in which the pipe is cut along a pattern by a laser cutting technology based on pattern data stored in a computer.

### "Method for providing coating layer on surface of stent main body, and method for providing covering layer on surface of stent main body"

A method may be adopted in which the surface of the linear member 2 constituting the stent main body is entirely or partly coated with a solution containing each of the above-mentioned compositions by use of a spray, a dispenser or ink jet capable of jetting a tiny quantity of material, and the like, followed by evaporating off the solvent. Or, a method may be adopted in which the stent main body is immersed in the above-mentioned solution, followed by evaporating off the solvent.

Means for expanding the stent 1 according to the present invention produced by the above-mentioned method is like common stents, and is not particularly limited. For instance, the stent may be of the self-expandable type, that is, the type in which when a force holding a stent folded into a tiny form is removed, the stent expands in the radial direction by its own restoring force. It is to be noted here, however, that the stent 1 according to the invention is preferably of the balloon-expandable type, that is, the type in which a balloon inside a stent main body is inflated so that the stent is expanded in the radial direction by the external force exerted by the balloon.

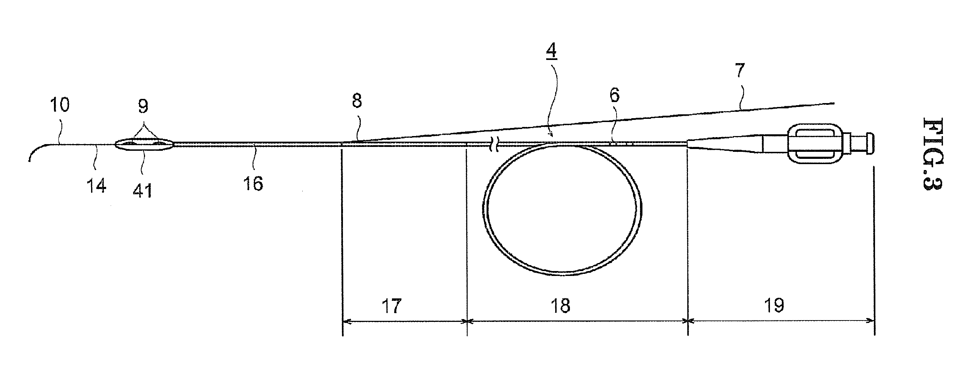

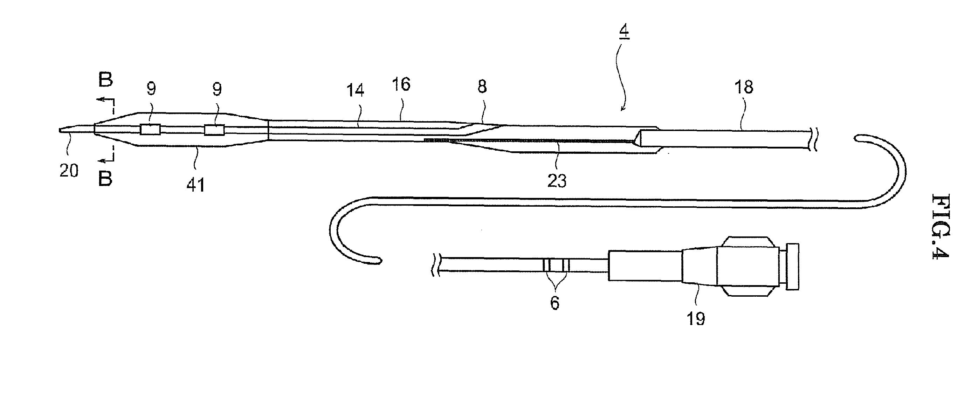

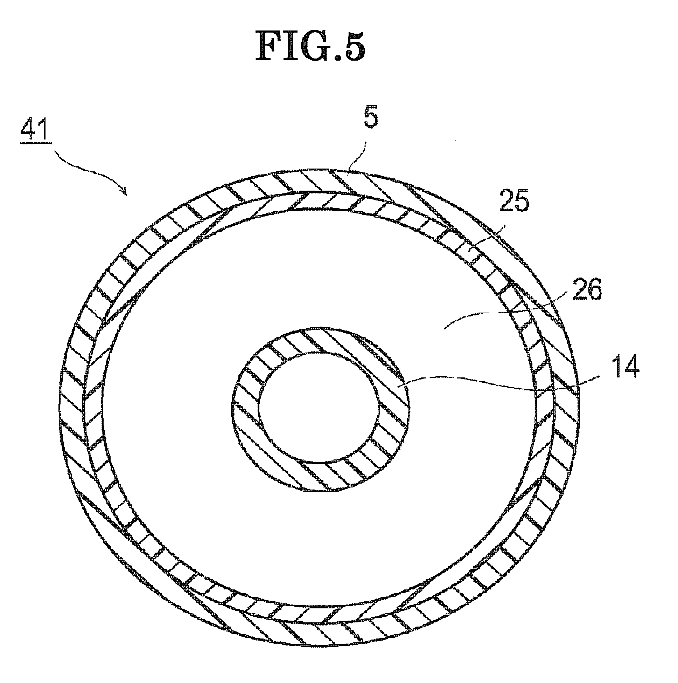

Now, a balloon catheter, which is a reference example, will be described in detail below. "Balloon catheter" FIG. 3 is a side view showing an embodiment of a balloon catheter, and FIG. 4 is a perspective view showing, in an enlarged form, major components of the balloon catheter of FIG. 3, by breaking a part of the balloon catheter and omitting a part of a proximal shaft 18. FIG. 5 is an enlarged cross-sectional view of a balloon taken along line B-B of FIG. 4. In general, balloon catheters are classified into two kinds, namely, the rapid exchange type and the over-the-wire type. FIGS. 3 and 4 show a rapid exchange type balloon catheter as an example of balloon catheter. Each of the components of the balloon catheter will be described more in detail below.

The balloon catheter 4 shown in FIGS. 3 and 4 is a so-called rapid exchange type catheter, and is inserted into a blood vessel along a guide wire 7, 10. The balloon catheter 4 composes a hub 19, a proximal shaft 18, an intermediate part 17, a distal shaft 16, a balloon 41, and an inner tube shaft 14, in this order from the proximal side.

The hub 19 on the proximal side is provided with a luer taper permitting connection to a pressure applying device such as an inflator. The proximal shaft 18 formed of a comparatively high-rigidity material such as metals and some resins is joined to the hub 19 in the manner of permitting fluid communication. The proximal shaft 18 is provided with depth markers 6 so that it is possible to easily check how deep the balloon catheter 4 is inserted into a guiding catheter (not shown) during angioplasty. As will be described in detail later, a distal portion of the proximal shaft 18 is made to be a reinforcement part 23, and an inflation aperture is provided in a part of a portion ranging from the reinforcement part to an unprocessed part. The inflation aperture is provided for permitting fluid communication between the inside and the outside of the proximal shaft 18. A fluid fed in under pressure through the hub 19 flows out from the inside of the proximal shaft 18 to the outside of the proximal shaft 18 (the inside of a lumen of the intermediate part 17) through the inflation aperture. Incidentally, a configuration may be adopted in which the inflation aperture is not provided, and a fluid passes through the inside of the reinforcement part from a proximal portion to a distal portion of the reinforcement part, and flows out to the outside of the reinforcement part (the inside of a lumen of the distal shaft 16).

The intermediate part 17 is provided on the distal side of the proximal shaft 18 so as to permit fluid communication. The distal shaft 16 formed of such a material as a resin and having a comparatively low rigidity is provided on the distal side of the intermediate part 17 so as to permit fluid communication. A proximal portion of the balloon 41 is provided on the distal side of the distal shaft 16 so as to permit fluid communication.

The inner tube shaft 14 coaxially penetrates the inside of the distal shaft 16 and the balloon 41. A distal portion of the inner tube shaft 14 is made to be a tip 20. The tip 20 is extended from the distal end of the balloon 41, and is joined to the distal side of the balloon 41 in a liquid-tight fashion. On the other hand, the proximal end of the inner tube shaft 14 is extended to a guide wire aperture 8 provided in a part of a portion ranging from the intermediate part 17 to the distal shaft 16, and is joined to the guide wire aperture 8 in a liquid-tight fashion. The guide wire 7, 10 shown in FIG. 3 is inserted into and passed through the inner tube shaft 14, with the distal opening of the tip 20 as an inlet and with the guide wire aperture 8 as an outlet. Radiopaque markers 9 are provided in the periphery of the inner tube shaft 14 inside the balloon 41.

When not inflated, the balloon 41 is in the state of being folded onto the outer periphery of the inner tube shaft 14. When inflated, the balloon 41 has its central portion put into a substantially hollow cylindrical shape, whereby a stenosed lesion of a blood vessel can be dilated easily. Incidentally, the central portion of the balloon 14 may not necessarily be put into a perfectly hollow cylindrical shape, and may be put into a polygonal prismatic shape. Besides, the radiopaque markers 9 are provided for facilitating positioning of the balloon 41 into a stenosed lesion under radioscopy during angioplasty.

While the balloon catheter shown in FIG. 3 or 4 has a double-wall tubular structure having the distal shaft 16 and the inner tube shaft 14, it may be a balloon catheter having a solid rod-like support member instead of the inner tube shaft. In addition, the balloon may be composed of a tubular balloon membrane 25 having a thickness of about 5 to 50 µm. A pressure fluid is led into or out of the balloon through a lumen 26, which is a space formed between the outside of the inner tube shaft and the inside of the distal shaft (see FIG. 5), whereby the balloon is inflated or contracted. Besides, the cross-section of the balloon has a hollow structure, as shown in FIG. 5, wherein the balloon membrane 25 is provided coaxially with the inner tube shaft 14 so as to surround the inner tube shaft 14. In this structure, further, it is possible that the surface of the balloon membrane 25 is covered with a layer 5 composed of the composition containing the medicinal agent, the medium-chain fatty acid triglyceride, and optionally the biodegradable polymer.

The material for the balloon membrane can be a material having a certain degree of plasticity so that a stenosed lesion of a blood vessel can be thereby dilated. Examples of the material which can be used here include such polymer materials as polyolefins (e.g., polyethylene, polypropylene, polybutene, ethylene-propylene copolymer, ethylene-vinyl acetate copolymer, ionomer), crosslinked products of polyolefin, polyesters (e.g., polyethylene terephthalate), polyester elastomers, polyvinyl chloride, polyurethane, polyurethane elastomers, polyphenylene sulfide, polyamides, polyamide elastomers, fluororesins, etc., silicone rubbers, latex rubbers, etc. In addition, laminate films obtained by appropriate lamination of these polymer materials can also be used. A configuration may be adopted in which a balloon 41 formed by biaxial orientation blow molding or the like is attached to the distal side of the distal shaft 16, and another configuration may be adopted in which orientation blow molding or the like is applied to a distal portion of the distal shaft 16 to form a balloon 41 integrally with the distal shaft 16.

The balloon can have an outside diameter of its hollow cylindrical part when inflated of 1.0 to 10 mm, preferably 1.0 to 5.0 mm, and a length thereof of 5 to 50 mm, preferably 10 to 40 mm, and an overall length of 10 to 70 mm, preferably 15 to 60 mm.

The thickness of the coating layer or covering layer 5 formed on a surface of the balloon catheter is set within such a range that the performances of the balloon such as deliverability to a lesion or stimulus to blood vessel walls are not spoiled and that the effect of release of the lipophilic medicinal agent is displayed sufficiently. The average thickness of the coating layer or covering layer 5 is preferably 0.01 to 10 µm, more preferably 0.1 to 10 µm, and further preferably 0.1 to 5 µm. Where the average thickness of the coating layer or covering layer pertaining to the present invention is 0.01 to 10 µm, the coating layer or covering layer can be uniformly formed on the whole part or a part of the surface of the balloon, and there is a low risk of breakage of the layer upon bending or inflation of the balloon.

In addition, the coating layer or covering layer 5 formed on a surface of the balloon catheter preferably covers 50 to 100% of the whole surface area of the balloon, and more preferably covers 80 to 100% of the whole surface area of the balloon.

### Examples

Now, the present invention will be described more in detail below by showing working examples, but the invention naturally is not limited to the examples.

### (Example 1)

In order to confirm the effect of addition of a medium-chain fatty acid triglyceride, a coating layer was formed on an SUS-made circular disc, and flexibility of the coating layer was evaluated.

A coating solution was prepared by dissolving 50 mg of sirolimus (Rapamycin, produced by SIGMA) which is an immunosuppressor as a biologically active agent, 50 mg of polylactic acid (PLA) (Resomer R207S, produced by Boehringer-Ingelheim) as a biodegradable polymer, and 10 mg of tricaprylin (capric acid triglyceride, produced by Wako Pure Chemical Industries, Ltd.) as a medium-chain fatty acid triglyceride in 1 mL of acetone. A SUS-made circular disc having a diameter of ϕ15 mm and a thickness of 0.5 mm was coated with the coating solution by use of a micro-dispenser. Thereafter, acetone was completely evaporated off by vacuum drying, to form a coating layer (a). The thickness of the coating layer (a) thus formed was 32 µm.

On the other hand, a SUS-made circular disc was coated with a coating solution prepared by dissolving 50 mg of sirolimus and 50 mg of PLA in 1 mL of acetone in the same manner as above, to form a coating layer (b) not containing a medium-chain fatty acid triglyceride. The thickness of the coating layer (b) thus formed was 30 µm.

In addition, a SUS-made circular disc was coated with a coating solution prepared by dissolving 50 mg of PLA and 10 mg (20% based on PLA) of tricaprylin in 1 mL of acetone in the same manner as above, to form a coating layer (c) not containing a biologically active agent. The thickness of the coating layer (c) thus formed was 17 µm.

As a reference, a SUS-made circular disc was coated with a coating solution prepared by dissolving 50 mg of PLA in 1 mL of acetone in the same manner as above, to form a coating layer (d) containing neither a biologically active agent nor a medium-chain fatty acid triglyceride. The coating layer (d) thus formed was 15 µm.

The circular discs provided thereon with the respective coating layers were sterilized by electron beams, and ultra-micro hardness under load (HTL) of the coating layers was measured according to JIS Z2255 by use of a dynamic ultra micro hardness tester produced by Shimadzu Corporation. A lower value of HTL indicates a higher flexibility of the coating layer. The results are shown in Table 1 below.

[Table 1]

**Table 1: Measurement results of ultra-micro hardness under load (HTL)**

| | **Biologically active agent** | **Biodegradable polymer** | **Medium-chain fatty acid triglyceride** | **HTL** |
|---|---|---|---|---|
| **Coating layer (a)** | **○** | **○** | **○** | **12** |
| **Coating layer (b)** | **○** | **○** | **×** | **20** |
| **Coating layer (c)** | **×** | **○** | **○** | **19** |
| **Coating layer (d)** | **×** | **○** | **×** | **24** |

| | | | | |
|---|---|---|---|---|
| **Notes -** ○**: Added, x: Not added** | | | | |

As a result, the coating layer formed by adding the biologically active agent and the medium-chain fatty acid triglyceride showed a remarkably enhanced flexibility. This results suggests that, where the coating layer formed by adding a medium-chain fatty acid triglyceride is provided on a stent in a covering fashion, the possibility of breakage of the coating layer at the time of expansion of the stent is low.

### (Example 2)

In order to examine the influence of the number of carbon atoms in the fatty acid constituting the medium-chain fatty acid triglyceride, a coating layer was formed on a SUS-made circular disc, and flexibility of the coating layer was evaluated.

Coating solutions were prepared by dissolving 50 mg of sirolimus which is an immunosuppressor as a biologically active agent, 50 mg of lactic acid-glycolic acid copolymer (PLGA) (Resomer RG756S, produced by Boehringer-Ingelheim) as a biodegradable polymer, and 10 mg (20% based on PLGA) of respective one of seven medium-chain fatty acid triglycerides shown in Table 2 in 1 mL of acetone.

Each of the coating solutions was applied to a SUS-made circular disc, followed by vacuum drying, to form respective coating layers, in the same manner as in Example 1. The respective coating layers had a thickness of about 30 µm.

The circular discs provided with the respective coating layers were sterilized by electron beams, and subjected to measurement of ultra-micro hardness under load (HTL) in the same manner as in Example 1. The results are shown in Table 2.

[Table 2]

**Table 2: Measurement results of ultra-micro hardness under load (HTL)**

| **Medium-chain fatty acid triglyceride** | **Number of carbon atoms in fatty acid** | **HTL** |
|---|---|---|
| **Triacetin** | **2** | **20** |
| **Tributyrin** | **4** | **19** |
| **Tricaproin** | **6** | **15** |
| **Tricaprylin** | **8** | **10** |
| **Caprylic acid/capric acid triglyceride (71/29)** | **8 and 10** | **10** |
| **Caprylic acid/capric acid triglyceride (57/43)** | **8 and 10** | **8** |
| **Tricaprin** | **10** | **9** |
| **Trilaurin** | **12** | **15** |
| **Trimyristin** | **14** | **20** |

| | | |
|---|---|---|
| **Notes -** **Triacetin: Acetic acid triglyceride, produced by Wako Pure Chemical Industries, Ltd.** **Tributyrin: Butyric acid triglyceride, produced by Wako Pure Chemical Industries, Ltd.** **Tricaproin: Caproic acid triglyceride, produced by Tokyo Chemical Industry Co., Ltd.** **Tricaprylin: Caprylic acid triglyceride, produced by Wako Pure Chemical Industries Ltd.** **Caprylic acid/capric acid triglyceride (71/29): content ratio of caprylic acid to capric acid = 71/29, Migliol 810, neutral oil produced by SASOL** **Caprylic acid/capric acid triglyceride (57/43): content ratio of caprylic acid to capric acid = 57/43, Migliol 812, neutral oil produced by SASOL** **Tricaprin: Capric acid triglyceride, produced by Tokyo Chemical Industry Co., Ltd.** **Trilaurin: Lauric acid triglyceride, produced by Tokyo Chemical Industry Co., Ltd.** **Trimyristin: Myristic acid triglyceride, produced by Tokyo Chemical Industry Col, Ltd.** | | |

As a result, the coating layers formed by adding the respective medium-chain fatty acid triglyceride consisted of a straight-chain saturated fatty acid of 6 to 12 carbon atoms showed remarkably enhanced flexibility.

### (Example 3)

In order to examine the influence of the addition amount of the medium-chain fatty acid triglyceride, coating layers were formed on SUS-made circular discs, and flexibility of the coating layers was evaluated.

Coating solutions were prepared by dissolving 50 mg of sirolimus which is an immunosuppressor as a biologically active agent, 50 mg of lactic acid-glycolic acid copolymer (PLGA) (Resomer RG756S, produced by Boehringer-Ingelheim) as a biodegradable polymer, and 0 mg, 2.5 mg, 5 mg, 10 mg, or 15 mg (0%, 5%, 10%, 20%, or 30% based on PLGA) of caprylic acid/capric acid triglyceride (57/43) as a medium-chain fatty acid triglyceride in 1 mL of acetone.

The coating solutions were respectively applied to SUS-made circular discs, followed by vacuum drying, to form coating layers, in the same manner as in Example 1. The coating layers had a thickness of about 30 to 38 µm.

The circular discs provided with the respective coating layers thereon were sterilized by electron beams, and subjected to measurement of ultra-micro hardness under load (HTL) in the same manner as in Example 1. The results are shown in Table 3.

[Table 3]

**Table 3: Measurement results of ultra-micro hardness under load (HTL)**

| **Addition amount of medium-chain fatty acid triglyceride** | **HTL** |
|---|---|
| **0%** | **20** |
| **5%** | **15** |
| **10%** | **10** |
| **20%** | **8** |
| **30%** | **8** |
| **60%** | **6** |

As a result, the coating layers started becoming flexible from an addition amount of 5%, and a substantially constant flexibility was resulted when the addition amount was 10% or more.

### (Example 4)

In order to examine the influence of the medium-chain fatty acid triglyceride on different compositional ratios of the biologically active agent and the biodegradable polymer, coating layers were respectively formed on SUS-made circular discs, and flexibility of the coating layers was evaluated.

Coating solutions were prepared by dissolving sirolimus which is an immunosuppressor as a biologically active agent, lactic acid-glycolic acid copolymer (PLGA) as a biodegradable polymer, and caprylic acid/capric acid triglyceride (57/43) as a medium-chain fatty acid triglyceride, in respective compositional ratios shown in Table 4, in 1 mL of acetone.

The coating solutions were respectively applied to SUS-made circular discs, followed by vacuum drying, to form coating layers, in the same manner as in Example 1. The coating layers had a thickness of about 30 µm.

The circular discs provided with the respective coating layers thereon were sterilized by electron beams, and subjected to measurement of ultra-micro hardness under load (HTL) in the same manner as in Example 1. The results are shown in Table 4.

[Table 4]

**Table 4: Measurement results of ultra-micro hardness under load (HTL)**

| **Biologically active agent** | **Bio-degradable polymer** | **Medium-chain fatty acid triglyceride** | **HTL** |
|---|---|---|---|
| **79** | **21** | **4** | **15** |
| **62** | **38** | **8** | **10** |
| **55** | **45** | **9** | **7** |
| **50** | **50** | **10** | **8** |
| **45** | **55** | **11** | **8** |
| **38** | **62** | **12** | **9** |
| **20** | **80** | **16** | **15** |

| | | | |
|---|---|---|---|
| **Note - Medium-chain fatty acid triglyceride was added in an amount of 20% based on biodegradable polymer.** | | | |

As a result, when the medium-chain fatty acid triglyceride was added in the condition where the compositional ratio of the biologically active agent and the biodegradable polymer was in the range from 20:80 to 80:20, the coating layers showed enhanced flexibility.

### (Example 5)

In order to examine the relationship between the flexibility of a coating layer and the breakage of the coating layer, a coating layer was actually formed on a stent, and the flexibility of the coating layer and the appearance of the coating layer when the stent was expanded were evaluated.

First, 50 mg of sirolimus (Rapamycin, produced by SIGMA) which is an immunosuppressor as a biologically active agent, 50 mg of polylactic acid (PLA) (Resomer R207S, produced by Boehringer-Ingelheim) as a biodegradable polymer, and 10 mg (20% base on PLA) of caprylic acid/capric acid triglyceride (71/29) (Migliol 810, a neutral oil produced by SASOL) were dissolved in 1 mL of acetone, to prepare a coating solution.

The coating solution was applied only to the outside surface of a linear member 2 (0.1 mm in width) constituting a stent main body (material: SUS316L) having a hollow cylindrical shape with an outside diameter of 2.0 mm, a thickness of 100 µm, and a length of 30 mm and having substantially rhombic cutouts, as shown in FIG. 1, by use of a micro-dispenser. Thereafter, acetone was completely evaporated off by vacuum drying, to form a coating layer. The average thickness of the coating layer thus formed was 13 µm.

The stent was crimped onto a balloon of a delivery catheter, and sterilized by electron beams. The ultra-micro hardness under load (HTL) of the coating layer after sterilization was measured according to JIS Z2255 by use of a dynamic ultra micro hardness tester produced by Shimadzu Corporation. As a result, the HTL was found to be 10.

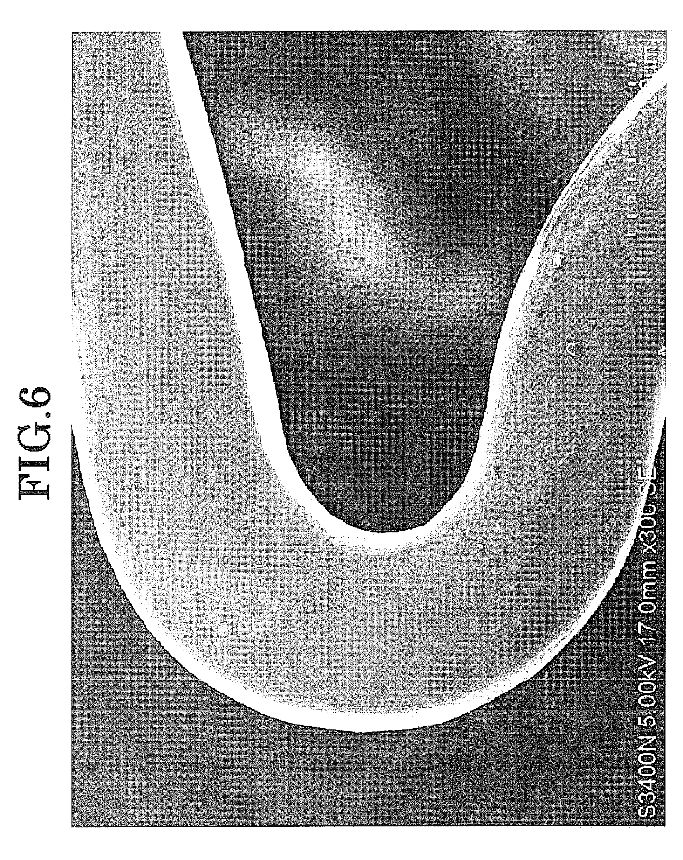

Thereafter, the balloon was inflated to expand the stent to an outside diameter of 3.0 mm, and the outside surface of the stent was observed under scanning electron microscope (SEM). As a result, damaged part such as cracking of the coating layer and exfoliation of the coating layer from the stent main body was not observed (see FIG. 6).

### (Example 6)

A coating solution was prepared by dissolving 50 mg of sirolimus (Rapamycin, produced by SIGMA) which is an immunosuppressor as a biologically active agent, 50 mg of lactic acid-glycolic acid copolymer (PLGA) (Resomer RG756S, produced by Boehringer-Ingelheim) as a biodegradable polymer, and 10 mg (20% based on PLGA) of caprylic acid/capric acid triglyceride (57/43) (Migliol 812, a neutral oil produced by SASOL) as a medium-chain fatty acid triglyceride in 1 mL of acetone. The coating solution was applied to a stent in the same manner as in Example 5, thereby forming a coating layer. The average thickness of the coating layer thus formed was 12 µm.

The stent was mounted onto a balloon of a delivery catheter, and sterilized by electron beams. Thereafter, ultra-micro hardness under load (HTL) of the coating layer was measured in the same manner as in Example 5. As a result, the HTL was found to be 8.

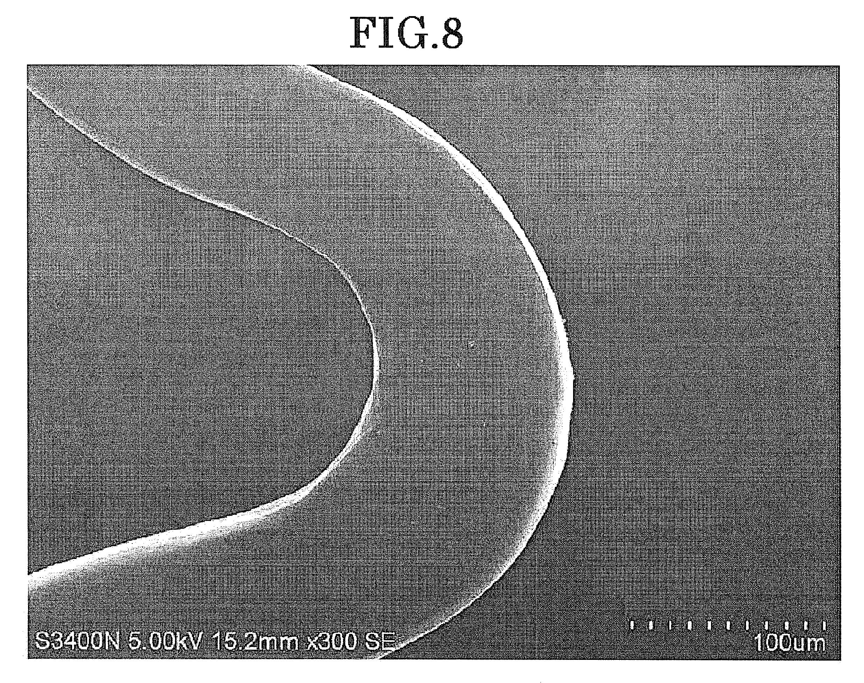

Thereafter, the balloon was inflated to expand the stent to an outside diameter of 3.0 mm, and the outside surface of the stent was observed under SEM. As a result, damaged part such as cracking of the coating layer or exfoliation of the coating layer from the stent main body was not observed (see FIG. 8).

### (Comparative Example 1)

A coating layer using sirolimus and polylactic acid was formed on a stent in the same conditions as in Example 5, except that a medium-chain fatty acid triglyceride was not added. The average thickness of the coating layer was 10 µm.

The stent was mounted on a balloon of a delivery catheter, and sterilized by electron beams. Thereafter, ultra-micro hardness under load (HTL) of the coating layer was measured in the same manner as in Example 5. The HTL was 21.

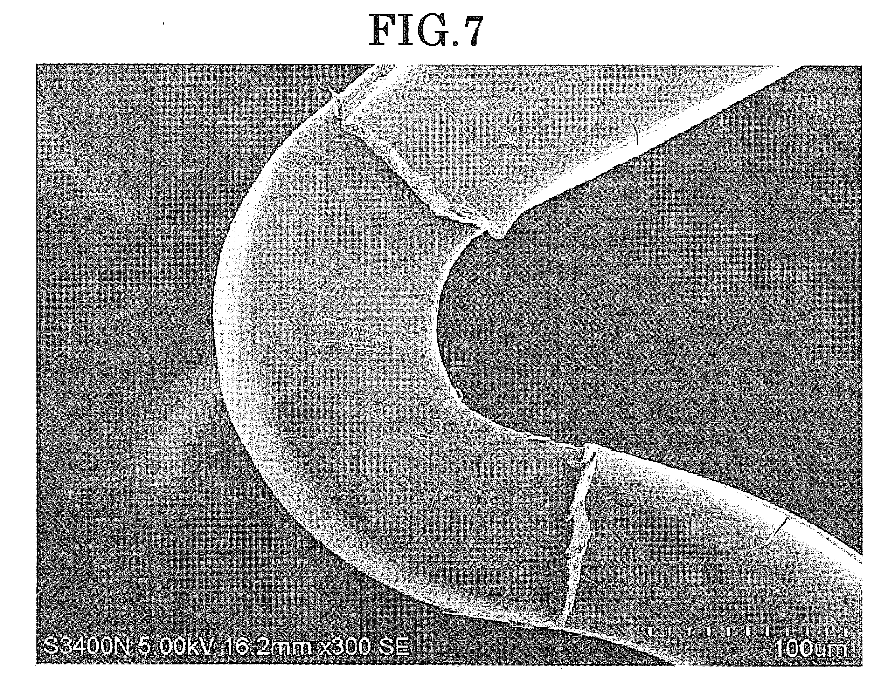

The stent was disposed (crimped) on the balloon of the delivery catheter, and the balloon was inflated to expand the stent to an outside diameter of 3.0 mm. Thereafter, the outside surface of the stent was observed under SEM. Damaged parts such as cracking of the coating layer and exfoliation of the coating layer from the stent main body, which are considered to be caused at the time of inflation of the balloon, were observed throughout the outside surface (see FIG. 7).

### (Comparative Example 2)

A coating layer using sirolimus and lactic acid-glycolic acid copolymer was formed on a stent in the same conditions as in Example 6, except that a medium-chain fatty acid triglyceride was not added. The average thickness of the coating layer was 10 µm.

The stent was mounted on a balloon of a delivery catheter, and sterilized by electron beams. Thereafter, ultra-micro hardness under load (HTL) of the coating layer on the stent was measured in the same manner as in Example 5. The HTL was 20.

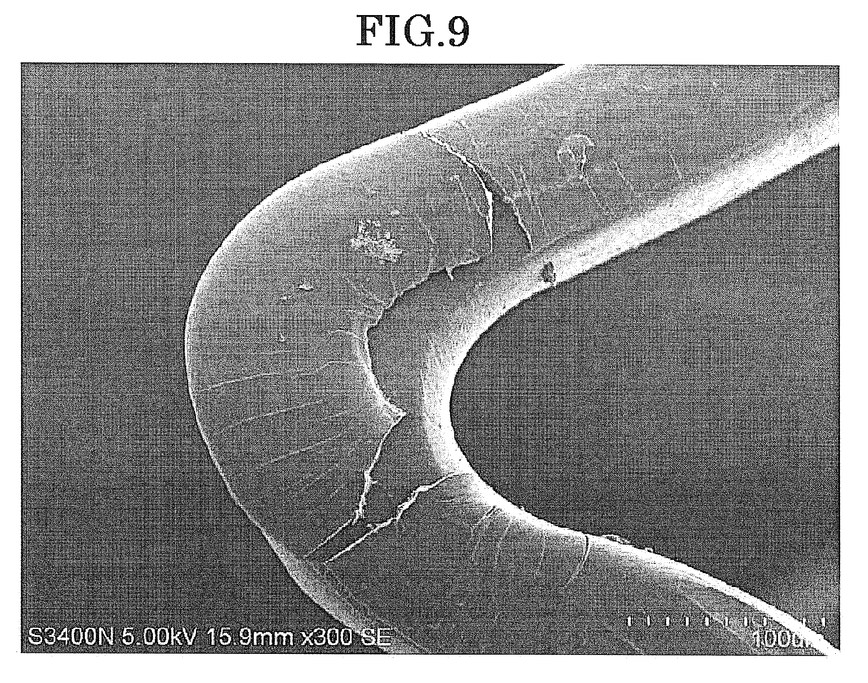

The stent was crimped on the balloon of the delivery catheter, and the balloon was inflated to expand the stent to an outside diameter of 3.0 mm. Thereafter, the outside surface of the stent was observed under SEM. As a result, damaged parts such as cracking of the coating layer and exfoliation of the coating layer from the stent main body, which are considered to be caused at the time of inflation of the balloon, were observed throughout the outside surface (see FIG. 9).

### (Examples 7 to 10, Comparative Example 3)

### (a) Production of drug eluting stent

As shown in Table 5 below, 36 mg of sirolimus (Rapamycin, produced by SIGMA) which is an immunosuppressor as a lipophilic medicinal agent, 50 mg of lactic acid-glycolic acid copolymer (PLGA) (Resomer RG756S, produced by Boehringer-Ingelheim) as a biodegradable polymer, and caprylic acid/capric acid triglyceride (57/43) (Migliol 812, a neutral oil produced by SASOL) as a medium-chain fatty acid triglyceride in respective amounts of 0 mg, 2.5 mg, 5 mg, 10 mg, and 15 mg (0% (Comparative Example 3), 5% (Example 7), 10% (Example 8), 20% (Example 9), and 30% (Example 10) by weight based on PLGA) were respectively dissolved in 1 mL of acetone, to prepare respective coating solutions.

These coating solutions were respectively applied to only the outside surface of a linear member 2 (0.1 mm in width) constituting a stent main body (material: SUS316L) having a hollow cylindrical shape with an outside diameter of 2.0 mm, a thickness of 100 µm, and a length of 30 mm and having substantially rhombic cutouts as shown in FIG. 1, by use of a micro-dispenser. Thereafter, acetone was completely evaporated off by vacuum drying, to form coating layers 3 as shown in FIG. 2. The thicknesses of the coating layers were 5 µm (Comparative Example 3), 5 µm (Example 7), 6 µm (Example 8), 6 µm (Example 9), and 7 µm (Example 10), respectively.

Each of the stents was crimped on a balloon of a delivery catheter (PTCA dilation catheter, produced by Terumo Corporation), and sterilized by electron beams.

The amounts of the medicinal agent carried, after the sterilization, were determined by HPLC (high performance liquid chromatography), to be 48 to 53 µm.

### (b) Evaluation of migration performance of medicinal agent to blood vessel tissue

Each of the drug eluting stents obtained in Examples 7 to 10 and Comparative Example 3 was tested for migration performance of the medicinal agent by a method described in Test 1 below, to examine the migration-accelerating ability of the medium-chain fatty acid triglyceride.

### Test 1

A rabbit having been perorally dosed with aspirin on the day before the treatment was anesthetized, and a 5-Fr sheath (introducer, produced by Terumo Corporation) was inserted into the carotid artery of the rabbit. Heparin was administered into the artery through the sheath, and a 5-Fr guiding catheter (PTCA guiding catheter, produced by Terumo Corporation) was inserted while feeding forward a guide wire (guide wire, produced by Terumo Corporation) in a preceding manner. The left and right iliac arteries were examined angiographically, and indwelling positions were determined. Thereafter, the delivery catheter with the drug eluting stent crimped thereon was inserted while feeding forward a PTCA guide wire (PTCA guide wire, produced by Terumo Corporation) in a preceding manner, and the balloon was inflated so as to expand the stent to an inside diameter of 3.0 mm. In this manner, one stent each was set indwelling in the left and right iliac arteries. After the lapse of 1 day, 7 days, and 28 days from the setting of the stent, the blood vessel in which the stent was left indwelling was extracted and washed in heparin-added saline. By use of a stereoscopic microscope, the outer membrane and the media of blood vessel were sampled from the blood vessel in which the stent had been put indwelling. The amounts of the medicinal agent contained in the outer membrane and the media thus sampled were determined by LC/MS (high performance liquid chromatography mass spectrometer). The values obtained by dividing the determined amounts of the medicinal agent by the weights of the outer membrane and the media served to determination, respectively, were defined as concentrations of the medicinal agent in the tissue.

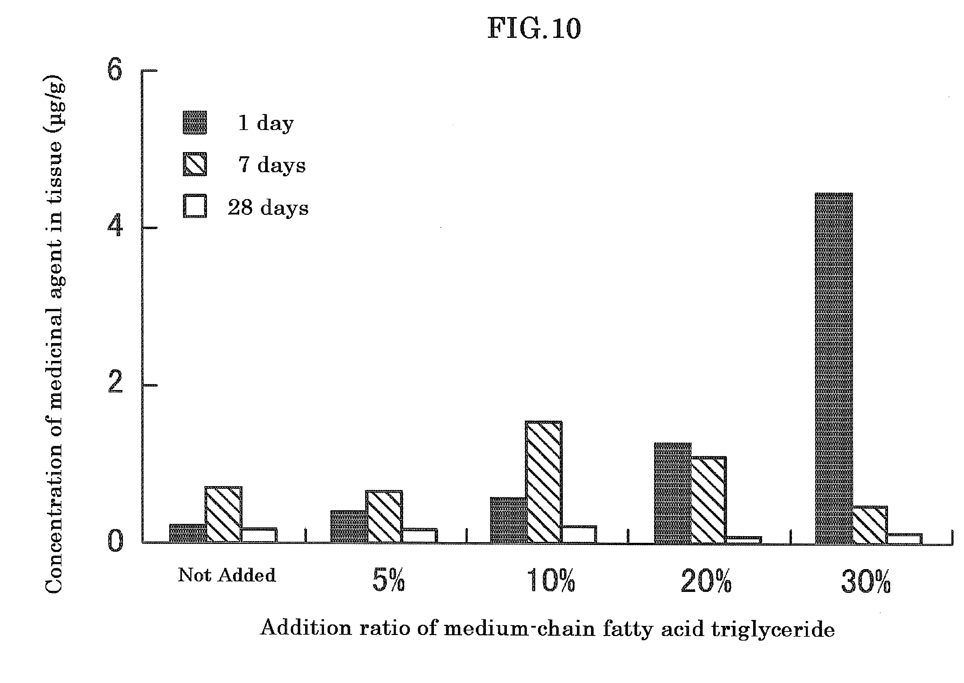

For the drug eluting stents, the concentrations of the medicinal agent in the tissue are collectively shown in Table 5 and FIG. 10.

[Table 5]

**Table 5: Measurement results of concentration of medicinal agent in tissue for drug eluting stent**

| | **Addition ratio of medium-chain fatty acid triglyceride (wt%)** | **Concentration of medicinal agent in tissue (µg/g)** | | |
|---|---|---|---|---|
| | | **1 day** | **7 days** | **28 days** |
| **Example 7** | **5%** | **0.40** | **0.69** | **0.18** |
| **Example 8** | **10%** | **0.58** | **1.57** | **0.27** |
| **Example 9** | **20%** | **1.30** | **1.12** | **0.11** |
| **Example 10** | **30%** | **4.41** | **0.50** | **0.16** |
| **Comparative Example 3** | **Not added** | **0.25** | **0.69** | **0.18** |

From Table 5 and FIG. 10, it is seen that the concentration of the medicinal agent in the tissue is raised as the addition ratio of the medium-chain fatty acid triglyceride increases. Particularly, the concentration of the medicinal agent in the tissue on the first day increases rapidly with an increase in the addition ratio. From this it is seen that the medium-chain fatty acid triglyceride shows an effect of migration of the medicinal agent into the tissue, from immediately after the contact of the coating layer in each of these Examples with the blood vessel tissue. On the other hand, it is seen that the concentration of the medicinal agent in the tissue on the seventh day reaches a maximum at an addition ratio of 10%, and decreases at addition ratios higher than 10%. This shows that the medium-chain fatty acid triglyceride has an effect of pushing out the medicinal agent having migrated into the cells, to the exterior of the cells. This suggests that when the addition ratio of the medium-chain fatty acid triglyceride becomes too high, it becomes impossible to maintain the effective concentration of the medicinal agent in the cells within the period necessary for suppressing restenosis.

### (c) Evaluation of effect of suppressing restenosis

For the drug eluting stents obtained in Examples 7 to 10 and Comparative Example 3, the effect of suppressing on restenosis was examined by the method shown in Test 2 below.

### Test 2

The drug eluting stents were set indwelling in the iliac arteries of a rabbit in the same manner as in Test 1. After 28 days of indwelling, the blood vessels in which the stents were left indwelling were extracted, and washed in heparin-added saline. Each of the blood vessels in which the stents were left indwelling was immobilized with formalin, dehydrated, immobilized with resin, and trisected into blood vessel pieces. Further, the blood vessel pieces were embedded in resin, and sliced sections were formed by use of a tungsten blade. Each of the resin-embedded sections was stained with hematoxylin and eosin stain (HE), and the cross-section of the blood vessel in which the stent had been indwelling was photographed by use of an optical microscope. The inside area of internal elastic layer and the inner membrane area were computed from the photograph image by use of an image analyzer, and occlusion area percentage ((inner membrane area)/(inside area of internal elastic layer) x 100) was computed.

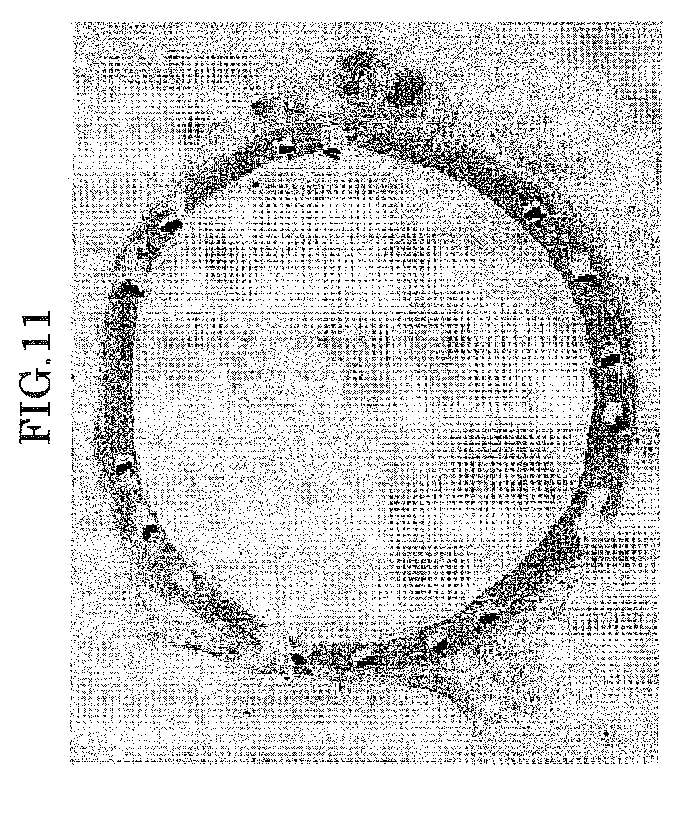

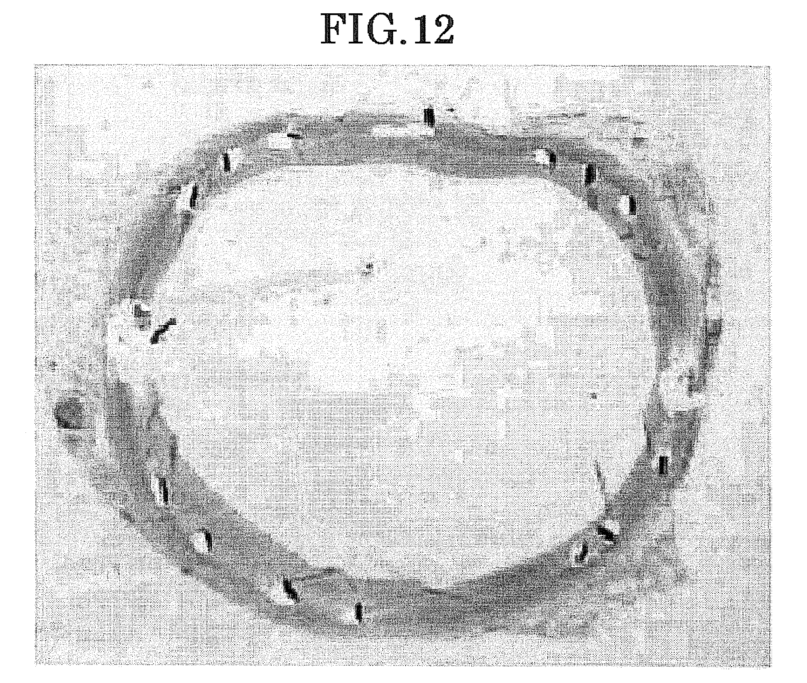

For the drug eluting stents, the occlusion area percentages are collectively set forth in Table 6. In addition, sectional photographs of pathological tissue specimens in Example 8 and Comparative Example 3 are shown in FIGS. 11 and 12.

[Table 6]

**Table 6: Measurement results of occlusion area percentage**

| | **Addition ratio of medium-chain fatty acid triglyceride (wt%)** | **Occlusion area percentage (%)** |
|---|---|---|
| **Example 7** | **5%** | **14.4** |
| **Example 8** | **10%** | **13.6** |
| **Example 9** | **20%** | **12.3** |
| **Example 10** | **30%** | **12.2** |
| **Comparative Example 3** | **Not added** | **17.1** |

From Table 6 it is seen that the occlusion area percentage is lower when the addition ratio of the medium-chain fatty acid triglyceride is in the range of 5 to 30% than when the triglyceride is not added, which in turn shows that restenosis rate is suppressed when the addition ratio is in this range. Thus, when the addition ratio of the medium-chain fatty acid triglyceride was in the just-mentioned range, a good effect of suppressing restenosis was exhibited, notwithstanding the amount of the medicinal agent carried on the stent was the same as that when the triglyceride was not added. This result suggests that the medium-chain fatty acid triglyceride enhanced the migration performance of the medicinal agent to such a level as to attain an in-tissue concentration effective in suppressing restenosis.

### (Reference Example 11, Comparative Example 4)

### (a) Production of drug-coated balloon catheter

As shown in Table 7, 100 mg of paclitaxel (Paclitaxel, produced by SIGMA) which is a carcinostatic agent as a medicinal agent, and 0 mg or 10 mg (respectively 0% or 20% based on the medicinal agent) of cparylic acid/capric acid triglyceride (71/29) (Migliol 810, a neutral oil produced by SASOL) as a medium-chain fatty acid triglyceride were dissolved in 1 mL of acetone, to prepare coating solutions.

These coating solutions were respectively applied to the outside surfaces of balloons 41 (balloons having a hollow cylindrical shape with an outside diameter of 3.0 mm and a length of 20 mm, material: polyamide) constituting balloon catheters (PTCA dilation catheter, produced by Terumo Corporation) shown in FIGS. 3 and 4 by dip coating by use of a dipping device. Thereafter, acetone was completely evaporated off by vacuum drying, to form coating layers 5 shown in FIG. 5. The thicknesses of the coating layers were 2 µm (Comparative Example 4) and 3 µm (Example 11). The balloon catheters were sterilized by electron beams. The amounts of the medicinal agent carried on the balloons after the sterilization were determined by HPLC (high performance liquid chromatography), to be 19 to 20 µg.

### (b) Evaluation of migration performance of medicinal agent into blood vessel tissue

For the drug-coated balloon catheters obtained in Reference Example 11 and Comparative Example 4, a test of drug migration performance was carried out by the method shown in Test 3 below, to examine the migration-accelerating ability of the medium-chain fatty acid triglyceride.

### Test 3

In the same manner as in Test 1, a 5-Fr guiding catheter (PTCA guiding catheter, produced by Terumo Corporation) was inserted into the iliac artery of a rabbit, and the left and right iliac arteries were angiographically imaged to determine balloon inflation positions. The drug-coated balloon catheter was inserted while feeding forward a PTCA guide wire (PTCA guide wire, produced by Terumo Corporation) in a preceding manner, the balloon was inflated so as to attain a balloon diameter of 3.0 mm, the inflation of the balloon was held at the balloon inflation position for 1 minute, and then the balloon catheter was pulled out. After 1 day, 7 days, and 28 days of indwelling, the blood vessels at the balloon inflation sites were extracted, and washed in heparin-added saline. Each of the blood vessels at the balloon inflation sites was trisected by use of a stereoscopic microscope, and the amount of the medicinal agent contained in the middle blood vessel piece was determined by LC/MS (high performance liquid chromatography mass spectrometer). A value obtained by dividing the determined amount by the weight of the blood vessel piece used for the determination was defined as the concentration of the medicinal agent in the tissue.

For the drug-coated balloon catheters, the concentrations of the medicinal agent in the tissue are shown in Table 7 below.

[Table 7]

**Table 7: Measurement results of concentration of medicinal agent in tissue for drug-coated balloon catheter**

| | **Addition ratio of medium-chain fatty acid triglyceride (wt%)** | **Concentration of medicinal agent in tissue (µg/g)** | | |
|---|---|---|---|---|
| | | **1 day** | **7 days** | **28 days** |
| **Example 11** | **20%** | **3.10** | **0.37** | **ND** |
| **Comparative** | **Not added** | **0.43** | **ND** | **ND** |
| **Example 4** | | | | |

| | | | | |
|---|---|---|---|---|
| **Note - ND: below detection limit** | | | | |

From Table 7 above, it is seen that addition of the medium-chain fatty acid triglyceride increases the concentration of the medicinal agent in the tissue. It was verified that the medium-chain fatty acid triglyceride permits efficient migration of the medicinal agent into the blood vessel tissue, notwithstanding the contact between the medicinal agent and the tissue is only momentary.

### Brief Description of Drawings

[FIG. 1]
   FIG. 1 is a side view showing an embodiment of a stent according to the present invention.
[FIG. 2]
   FIG. 2 is an enlarged cross-sectional view taken along line A-A of FIG. 1.
[FIG. 3]
   FIG. 3 is a side view showing an embodiment of a balloon catheter according to the invention.
[FIG. 4]
   FIG. 4 is a side view showing an embodiment of a balloon catheter.
[FIG. 5]
   FIG. 5 is an enlarged cross-sectional view taken along line B-B of FIG. 4.
[FIG. 6]
   FIG. 6 is an SEM photograph of a surface of a stent after expansion in Example 5.
[FIG. 7]
   FIG. 7 is an SEM photograph of a surface of a stent after expansion in Comparative Example 1.
[FIG. 8]
   FIG. 8 is an SEM photograph of a surface of a stent after expansion in Example 6.
[FIG. 9]
   FIG. 9 is an SEM photograph of a surface of a stent after expansion in Comparative Example 2.
[FIG. 10]
   FIG. 10 shows measurement results of the concentration of a medicinal agent in a tissue in Examples 7 to 10 and Comparative Example 3.
[FIG. 11]
   FIG. 11 is a cross-sectional photograph of a pathological tissue sample in Example 8.
[FIG. 12]
   FIG. 12 is a cross-sectional photograph of a pathological tissue sample in Comparative Example 3.

### Description of Reference Symbols

- 1: Stent (Stent main body)
- 11: Substantially rhombic element
- 12: Annular unit
- 13: Link member
- 2: Linear member
- 21: Outside surface
- 22: Inside surface
- 3: Coating layer or covering layer
- 4: Balloon catheter
- 6: Depth marker
- 7, 10: Guide wire
- 8: Guide wire aperture
- 9: Radiopaque marker
- 14: Inner tube shaft
- 16: Distal shaft
- 17: Intermediate part
- 18: Proximal shaft
- 19: Hub
- 20: Tip
- 23: Reinforcement part
- 25: Balloon membrane
- 26: Lumen
- 41: Balloon
- 5: Coating layer or covering layer

## Claims

1. A stent to be put indwelling in a lumen in vivo, comprising a stent main body, and a layer composed of a composition containing a biologically active agent, a biodegradable polymer, and a medium-chain fatty acid triglyceride, the layer being formed on at least a part of a surface of the stent main body,
wherein the medium-chain fatty acid triglyceride is defined by the following chemical formula 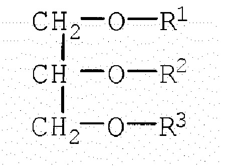 wherein R¹, R², and R³ are each independently a straight-chain acyl group of 6 to 12 carbon atoms, and
wherein the addition amount of said medium-chain fatty acid triglyceride in the layer is at least 5 % based on the biodegradable polymer.

2. The stent according to claim 1, wherein the medium-chain fatty acid triglyceride is a triglyceride synthesized from glycerin and at least one straight-chain saturated fatty acid selected from the group consisting of caprylic acid, capric acid, and a mixture of caprylic acid and capric acid.

3. The stent according to any of claims 1 or 2, wherein the medium-chain fatty acid triglyceride is contained in the composition in an amount of 5 to 60 wt% based on the biodegradable polymer.

4. The stent according to any of claims 1 to 3, wherein the biodegradable polymer is at least one polymer selected from the group consisting of polyester, aliphatic polyester, polyacid anhydride, polyorsoester, polycarbonate, polyphosphazene, polyphosphate ester, polyvinyl alcohol, polypeptide, polysaccharide, protein, and cellulose, a copolymer obtained by arbitrary copolymerization of monomers constituting said polymers, or a mixture of said polymer(s) and/or said copolymer(s).

5. The stent according to any of claims 1 to 4, wherein the biodegradable polymer is an aliphatic polyester.

6. The stent according to any of claims 1 to 5, wherein the biologically active agent is at least one compound selected from the group consisting of carcinostatic agent, immunosuppressor, antibiotic, antirheumatic, antithrombotic agent, HMG-CoA reductase inhibitor, ACE inhibitor, calcium antagonist, antilipemia agent, integrin inhibitor, antiallergic agent, antioxidant, GPIIbIIIa antagonist, retinoid, flavonoid, carotenoid, lipid improver, DNA synthesis inhibitor, tyrosine kinase inhibitor, antiplatelet agent, antiinflammatory agent, bio-derived material, interferon, and NO production accelerating substance.

7. The stent according to claim 1, which is a drug eluting stent.

8. The stent according to claim 1, wherein the stent main body is formed from a metal or a polymer.

## Patentansprüche

1. Stent, welcher verweilend in ein Lumen in vivo eingesetzt werden soll, umfassend einen Stent-Hauptkörper, und eine Schicht zusammengesetzt aus einer Zusammensetzung, welche ein biologisch aktives Agens, ein biologisch abbaubares Polymer und ein mittelkettiges Fettsäuretriglycerid enthält, wobei die Schicht auf mindestens einem Teil einer Oberfläche des Stent-Hauptkörpers ausgebildet ist,
wobei das mittelkettige Fettsäuretriglycerid durch die folgende chemische Formel definiert ist: 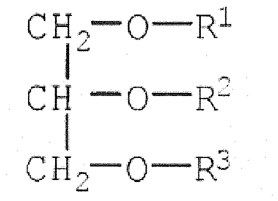 wobei R¹, R² und R³ jeweils unabhängig voneinander eine geradkettige Acylgruppe von 6 bis 12 Kohlenstoffatomen ist, und
wobei die Zugabemenge des mittelkettigen Fettsäuretriglycerids in der Schicht mindestens 5% beträgt, basierend auf dem biologisch abbaubaren Polymer.

2. Stent gemäß Anspruch 1, wobei das mittelkettige Fettsäuretriglycerid ein Triglycerid ist, welches aus Glycerin und mindestens einer geradkettigen gesättigten Fettsäure, ausgewählt aus der Caprylsäure, Caprinsäure und einer Mischung aus Caprylsäure und Caprinsäure bestehenden Gruppe, synthetisiert ist.

3. Stent gemäß einem der Ansprüche 1 oder 2, wobei das mittelkettige Fettsäuretriglycerid in der Zusammensetzung in einer Menge von 5-60 Gew.-% basierend auf dem biologisch abbaubaren Polymer enthalten ist.

4. Stent gemäß einem der Ansprüche 1 bis 3, wobei das biologisch abbaubare Polymer mindestens ein Polymer, das aus der Gruppe aus Polyester, aliphatischem Polyester, Polysäure-Anhydrid, Polyorsoester, Polycarbonat, Polyphosphazen, Polyphosphatester, Polyvinylalkohol, Polypeptid, Polysaccharid, Protein und Cellulose ausgewählt ist, ein Copolymer, das durch eine beliebige Copolymerisierung von Monomeren, welche besagte Polymere aufbauen, erhalten wurde, oder eine Mischung des/der Polymer(e) und/oder des/der Copolymer(e) ist.

5. Stent gemäß einem der Ansprüche 1 bis 4, wobei das biologisch abbaubare Polymer ein aliphatisches Polyester ist.

6. Stent gemäß einem der Ansprüche 1 bis 5, wobei das biologisch aktive Agens mindestens eine Verbindung aus der aus carzinostatischem Agens, Immunsuppressor, Antibiotikum, Anti-Rheumatikum, Anti-Thrombosemittel, HMG-CoA- Reduktase-Inhibitor, ACE-Inhibitor, Calcium-Antagonist, anti-lipämischem Agens, Integrin-Inhibitor, anti-allergischem Agens, Antioxidans, GPIIbIIIa-Antagonist, Retinoid, Flavonoid, Carotinoid, Lipid-Verbesserer, DNA-Synthese-Inhibitor, Thyrosin-Kinase-Inhibitor, thrombozytenaggregationshemmendem Agens, anti-entzündlichem Agens, bio-abgeleitetem Material, Interferon und einer NO-Produktions-beschleunigenden Substanz bestehenden Gruppe ist.

7. Stent gemäß Anspruch 1, welcher ein Wirkstoffeluierender Stent ist.

8. Stent gemäß Anspruch 1, wobei der Stent-Hauptkörper aus einem Metall oder Polymer gebildet ist.

## Revendications

1. Stent à installer à demeure dans une lumière *in vivo,* comprenant un corps principal de stent et une couche composée d'une composition contenant un agent biologiquement actif, un polymère biodégradable, et un triglycéride d'acide gras à chaîne moyenne, la couche étant formée sur au moins une partie d'une surface du corps principal de stent,
dans lequel le triglycéride d'acide gras à chaîne moyenne est défini par la formule chimique suivante : 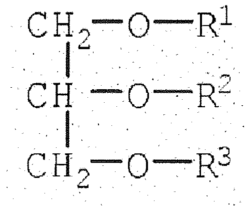 dans laquelle R¹, R², et R³ sont chacun indépendamment un groupe acyle à chaîne linéaire ayant 6 à 12 atomes de carbone, et
dans lequel la quantité d'ajout dudit triglycéride d'acide gras à chaîne moyenne dans la couche est d'au moins 5% sur la base du polymère biodégradable.

2. Stent selon la revendication 1, dans lequel ledit triglycéride d'acide gras à chaîne moyenne est un triglycéride synthétisé à partir de glycérine et d'au moins un acide gras saturé à chaîne linéaire sélectionné dans le groupe constitué par l'acide caprylique, l'acide caprique, et un mélange d'acide caprylique et d'acide caprique.

3. Stent selon l'une quelconque des revendications 1 à 2, dans lequel le triglycéride d'acide gras à chaîne moyenne est contenu dans la composition en une quantité de 5 à 60% en poids sur la base du polymère biodégradable.

4. Stent selon l'une quelconque des revendications 1 à 3, dans lequel le polymère biodégradable est au moins un polymère sélectionné dans le groupe constitué par un polyester, un polyester aliphatique, un anhydride de polyacide, un polyorthoester, un polycarbonate, un polyphosphazène, un ester de polyphosphate, un alcool polyvinylique, un polypeptide, un polysaccharide, une protéine, et une cellulose, un copolymère obtenu par copolymérisation aléatoire de monomères constituant lesdits polymères, ou un mélange dudit ou desdits polymère(s) et/ou dudit ou desdits copolymère(s).

5. Stent selon l'une quelconque des revendications 1 à 4, dans lequel le polymère biodégradable est un polyester aliphatique.

6. Stent selon l'une quelconque des revendications 1 à 5, dans lequel l'agent biologiquement actif est au moins un composé sélectionné dans le groupe constitué par un agent carcinostatique, un immunosuppresseur, un antibiotique, un agent antirhumatismal, un agent antithrombotique, un inhibiteur de la HMG-CoA réductase, un inhibiteur de l'ECA, un antagoniste calcique, un agent hypolipémiant, un inhibiteur de l'intégrine, un agent antiallergique, un antioxydant, un antagoniste de GPIIbIIIa, un rétinoïde, un flavonoïde, un caroténoïde, un agent améliorant le statut lipidique, un inhibiteur de la synthèse de l'ADN, un inhibiteur de la tyrosine kinase, un antiplaquettaire, un agent anti-inflammatoire, une substance d'origine biologique, un interféron, et une substance d'accélération de la production de NO.

7. Stent selon la revendication 1, qui est un stent à élution médicamenteuse.

8. Stent selon la revendication 1, dans lequel le corps principal de stent est formé à partir d'un métal ou d'un polymère.
